(19) Europäisches Patentamt
European Patent Office
Office européen des brevets

(11) **EP 2 411 500 B1**

(12) **FASCICULE DE BREVET EUROPEEN**

(45) Date de publication et mention de la délivrance du brevet:
**28.03.2018   Bulletin 2018/13**

(51) Int Cl.:
*C12M 1/00* *(2006.01)*          *C12M 1/12* *(2006.01)*
*C12N 1/12* *(2006.01)*

(21) Numéro de dépôt: **10714930.4**

(22) Date de dépôt: **09.03.2010**

(86) Numéro de dépôt international:
**PCT/FR2010/050395**

(87) Numéro de publication internationale:
**WO 2010/109108 (30.09.2010 Gazette 2010/39)**

(54) **Procédé pour la culture de microorganismes photosynthétiques**

Verfahren zur Kultivierung von photosynthetischen Mikroorganismen

Method for cultivating photosynthetic microorganisms

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **25.03.2009   FR 0951917**

(43) Date de publication de la demande:
**01.02.2012   Bulletin 2012/05**

(73) Titulaire: **Microphyt**
**34670 Baillargues (FR)**

(72) Inventeur: **MULLER-FEUGA, Arnaud**
**F-34670 Baillargues (FR)**

(74) Mandataire: **Chevalier, Renaud Philippe**
**Cabinet Germain & Maureau**
**BP 6153**
**69466 Lyon Cedex 06 (FR)**

(56) Documents cités:
**EP-A- 1 925 660     FR-A- 2 685 344**
**FR-A- 2 914 315     US-A1- 2007 231 886**

• **MULLER-FEUGA A ET AL: "Swirling Flow Implementation in a Photobioreactor for Batch and Continuous Cultures of Porphyridium cruentum" BIOTECHNOLOGY AND BIOENGINEERING, WILEY & SONS, HOBOKEN, NJ, US, vol. 84, no. 5, 5 décembre 2003 (2003-12-05), pages 544-551, XP002539240 ISSN: 0006-3592 [extrait le 2003-09-24]**
• **MULLER-FEUGA ET AL.: "Comparison of artificial ligth photobioreactors and other production systems using Porphydium cruentum", JOURNAL OF APPLIED PHYCOLOGY, vol. 10, no. 1, 1 February 1998 (1998-02-01), pages 83-90,**
• **MOLINA ET AL.: "Tubular photobioreactor design for algal cultures", JOURNAL OF BIOTECHNOLOGY, vol. 92, 28 December 2001 (2001-12-28), pages 113-131,**

**Description**

La présente invention se rapporte à un procédé de culture de microorganismes photosynthétiques utilisant un réacteur. Le procédé se réalise dans un réacteur photosynthétique comprenant :

- au moins une canalisation de réaction photosynthétique dans laquelle circule le milieu de culture liquide et qui est pourvue d'au moins un tronçon de réaction sensiblement horizontal et réalisé au moins partiellement dans un matériau transparent au rayonnement solaire, ladite canalisation présentant une extrémité basse disposée en partie basse du réacteur et une extrémité haute disposée en partie haute du réacteur au-dessus de l'extrémité basse ;
- au moins une canalisation de retour assurant la liaison fluidique entre l'extrémité basse et l'extrémité haute de la canalisation de réaction ; et
- au moins un moyen de mise en circulation du milieu de culture liquide.

La présente invention s'applique à la culture de tout organisme photosynthétique, c'est-à-dire de toute forme de vie susceptible de développement et de photosynthèse dans un milieu de culture nutritif approprié, en présence de rayonnement solaire et de gaz riche en carbone, tel que du dioxyde de carbone.

Parmi les microorganismes photosynthétiques concernés par l'invention figurent plus particulièrement les végétaux aquatiques comme par exemple les micro-algues, les protonémas de mousse, les petites macro-algues et les cellules isolées de plantes multicellulaires. Ces végétaux aquatiques présentent des propriétés intéressantes dans les domaines notamment de la pharmacie, de la nutrition humaine et animale, de la dermo-cosmétologie, de l'énergie et de l'environnement.

Comme pour la plupart des microorganismes photosynthétiques, l'accès à cette ressource consiste essentiellement dans la culture assistée dans des réacteurs adaptés. La lumière étant leur principal substrat, le milieu de culture doit présenter une interface optique recevant un flux de lumière. La difficulté de cultiver des microorganismes photosynthétiques tient au fait qu'ils constituent eux-mêmes des obstacles au passage de la lumière qui est leur principal substrat. La croissance de la culture va donc se stabiliser lorsque la lumière ne pénètrera plus dans l'épaisseur de la culture. Ce phénomène est appelé l'auto-ombrage.

La longueur du chemin optique, ou « light path length », permet de caractériser les différents modes de confinement, et se définit comme étant :

- la longueur du parcours de la lumière depuis son entrée dans la culture par une interface optique transparente jusqu'à une paroi opaque opposée ; ou
- la moitié de la distance qui sépare les deux interfaces optiques transparentes lorsque le confinement reçoit la lumière par deux interfaces optiques transparentes opposées.

Cette longueur du chemin optique varie entre quelques centimètres et quelques décimètres et détermine pour l'essentiel la production de biomasse par unité de temps et de surface optique (productivité surfacique en $g/m^2/j$) et la concentration de la culture (en g/L) en phase finale de croissance. Les différents modes de confinement qui sont mis en oeuvre pour assurer la culture des petits végétaux aquatiques peuvent ainsi être classés en fonction de cette longueur caractéristique.

La réaction de photosynthèse s'accompagne également d'une consommation de gaz carbonique ($CO_2$) et d'une production d'oxygène ($O_2$). L'excès d'oxygène inhibe la réaction, tandis que l'absence de gaz carbonique l'interrompt par défaut de substrat à transformer. Une interface gaz/liquide doit donc être aménagée pour les transferts de masse entre ces gaz et la phase liquide. Afin de favoriser ces échanges et d'éviter les hétérogénéités, la culture doit être le siège d'un mélange destiné à renouveler les organismes au niveau de l'interface optique susmentionnée et également au niveau de cette interface gaz/liquide.

Un premier mode de réalisation connu de réacteur photosynthétique consiste en un récipient ouvert de type bassin ou bac où la culture est maintenue par gravité et présente une surface libre réalisant à elle seule l'interface optique et l'interface liquide/gaz. La culture est mélangée à l'intérieur du bassin par un ou plusieurs dispositifs mécaniques de brassage, par exemple de type roue à aube. Les cultures en bassin ainsi réalisées peuvent couvrir des surfaces importantes et cette forme de réalisation est à l'origine de l'essentiel de la production mondiale actuelle de microalgues, qui atteint plusieurs milliers de tonnes de poids sec. Les organismes photosynthétiques produits par ce type de réacteur sont essentiellement :

- des algues dites extrêmophiles dont les milieux sont hostiles aux prédateurs et aux compétiteurs, comme par exemple les algues du type spiruline ou Dunaliella ; ou
- des algues dites dominantes qui supportent les sollicitations mécaniques ou les contaminations mieux que les autres, comme par exemple les algues du type Chlorella, Scenedesmus, Skeletonema, Odontella ou Nannochloropsis.

Un second mode de réalisation connu de réacteur photosynthétique consiste également en un récipient ouvert de type réservoir ou bac mais dont les dimensions sont inférieures à celles des bassins du premier mode de réalisation connu. Ces récipients présentent généralement des parois latérales transparentes au rayonnement solaire, de sorte que l'interface optique est constituée à la fois par la surface libre du milieu liquide et par les parois latérales transparentes.

Dans ce second mode de réalisation, il est classique de recourir à une injection d'air effectuée dans la partie basse du réservoir qui conduit à la formation de bulles d'air remontant dans le liquide jusqu'à la surface libre. La surface des bulles ainsi formées constitue l'interface gaz/liquide. En remontant vers la surface, les bulles entraînent la culture vers le haut, créant ainsi des mouvements convectifs qui peuvent s'étendre à tout le volume. Du dioxyde de carbone ($CO_2$) est parfois ajouté à l'air injecté pour apporter un surcroît de carbone selon une fraction molaire prédéfinie de quelques pourcents.

De volume inférieur à celui des bassins du premier mode de réalisation, les réservoirs du second mode de réalisation connu sont adaptés aux cultures plus contrôlées, en particulier aux cultures de microalgues destinées à la nutrition des larves de mollusques ou des proies vivantes de larves de poissons en aquaculture. Le nettoyage fréquent de ces réservoirs ainsi que des inoculations pures et massives permettent de limiter les contaminations à l'intérieur du réservoir. Au nombre de plusieurs dizaines d'espèces les microalgues ainsi cultivées présentent des besoins en température et en lumière relativement voisins qui rendent possible leur culture dans des locaux communs.

Ces deux modes de réalisation sous la forme d'un récipient ouvert offrent une longueur de chemin optique de un à plusieurs décimètres.

Un troisième mode de réalisation connu de réacteur photosynthétique consiste en un réacteur clos dit photobioréacteur et qui comprend une boucle fermée à l'intérieur de laquelle circule le milieu de culture liquide, ladite boucle fermée comprenant une canalisation de réaction pourvue de tronçons de réaction réalisés dans un matériau transparent au rayonnement lumineux (ou à la lumière), et une canalisation de retour assurant la liaison entre les deux extrémités opposées de la canalisation de réaction.

Les photobioréacteurs, décrits notamment dans les documents GB 2 118 572 A, ES 2 193 860 A1, GB 2 331 762 A, ES 2 150 389 A1, FR 2 685 344 A1 et FR 2 875 511 A3, offrent des longueurs de chemin optique sensiblement plus faibles, de l'ordre de un à plusieurs centimètres, par rapport aux modes de réalisation avec récipient ouvert, et ils permettent d'atteindre des concentrations en organismes photosynthétiques de plusieurs grammes par litre à l'abri des contaminations aériennes. La canalisation de réaction des photobioréacteurs consiste généralement en des plaques ou tubes transparents, en verre ou matière plastique, d'épaisseur ou de diamètre de l'ordre du centimètre, qui sont connectés bout à bout par des coudes pour former ensemble une canalisation en forme de serpentin.

La canalisation de retour comprend un tube vertical dit ascendant, dans lequel le milieu liquide remonte, et un tube vertical descendant dans lequel le milieu liquide descend sous l'effet de la gravité.

Le système d'injection de gaz généralement mis en oeuvre dans les photobioréacteurs consiste en un gazosiphon, autrement appelé « gas-lift » ou dispositif d'ascenseur à gaz, c'est-à-dire en une injection de gaz à la base du tube vertical ascendant de la canalisation de retour ; ladite injection de gaz servant à la fois à mettre en circulation ou déplacer le milieu réactionnel liquide et à réaliser les échanges gaz-liquide. Le gazosiphon comporte en partie haute un réservoir de charge ou volume élargi dans lequel les vitesses de circulation plus faibles permettent la séparation gaz-liquide, et le tube vertical descendant de la canalisation de retour débouche dans le fond du réservoir de charge pour alimenter en liquide la canalisation de réaction.

Les photobioréacteurs susmentionnés appliquent le principe selon lequel la réaction n'a lieu que dans la phase liquide, autrement dit ces photobioréacteurs cherchent à minimiser le volume de gaz injecté dans le réacteur pour ne pas diminuer d'autant le volume du milieu de culture liquide, dans un souci de ne pas faire diminuer la production. Ainsi, dans ces photobioréacteurs, l'extraction d'oxygène est réalisée par le moyen du tube ascendant vertical défini ci-dessus ; ledit tube ascendant vertical formant une colonne à bulles d'air débouchant dans le réservoir de charge recevant le milieu de culture liquide, et comportant une injection de gaz en partie basse, opportunément de l'air enrichi de CO2. Comme décrit ci-dessus, les deux fonctions de circulation et de transfert gazeux sont confondues au sein de cet unique dispositif, appelé gazosiphon, qui crée une circulation verticale ascendante par échange de quantité de mouvement entre la masse liquide et les bulles de gaz résultant de l'injection. L'oxygène photosynthétique en sursaturation dans le liquide passe dans la phase gazeuse par balayage à l'air, tandis que le $CO_2$ passe en solution. Ces fonctions de dégazage et de carbonatation sont indispensables et interviennent simultanément au niveau de cet unique dispositif.

Les gazosiphons présentent l'inconvénient de générer des bulles de gaz qui remontent dans le tube ascendant vertical de la canalisation de retour des photobioréacteurs. La demanderesse a en effet observé le rôle délétère de ces bulles pour la culture de microorganismes dans les photobioréacteurs :

- d'une part, les bulles sollicitent mécaniquement les microalgues et peuvent nuire à des microrganismes fragiles ; et
- d'autre part, les bulles captent par effet tensio-actif les molécules qui présentent des propriétés surfactantes, et notamment les molécules organiques, débris cellulaires et les produits d'excrétion des cellules vivantes. Ces substances, normalement dispersées dans le milieu en l'absence de bulles, sont ainsi rassemblées sous forme d'agrégats à la surface libre du réservoir de charge lorsque les bulles éclatent. Les bactéries et champignons qui ne pourraient se développer en raison de la forte dilution de ces molécules organiques trouvent alors des substrats concentrés favorables à leur développement.

L'un des buts de la présente invention est d'éviter, ou du moins limiter, la formation des bulles pour :

- contenir le développement bactérien et fongique, par exemple pour rester compatible avec les normes sanitaires classiquement imposées dans la culture de microorganismes ; et pour
- limiter les sollicitations mécaniques dans le milieu de culture liquide, et ainsi permettre la culture de certains microorganismes fragiles qui étaient jusqu'à là exclus d'une telle culture en réacteur.

Dans une réalisation alternative au gazosiphon, la désoxygénation du milieu de culture liquide circulant dans le photobioréacteur est obtenue en faisant chuter gravitationnellement le milieu liquide dans un récipient à niveau constant. Le milieu de culture liquide est ici mis en circulation par un moyen de pompage, notamment du type pompe centrifuge, disposé dans la canalisation de réaction conçu pour non seulement compenser les pertes de charge dans la canalisation mais aussi élever la culture de la hauteur de la chute.

Bien que moins générateur de bulles, ce dispositif avec pompe centrifuge est aussi mécaniquement dommageable pour les microorganismes que le gazosiphon. En effet, pour vaincre les pertes de charge, il y a génération à chaque passage au droit du moyen de pompage d'efforts mécaniques qui peuvent contrarier la croissance des microorganismes et provoquer des mortalités au sein de la culture. Les performances de production s'en trouvent alors altérées, parfois de façon rédhibitoire.

Par exemple, on a constaté qu'il n'est pas possible de cultiver certaines microalgues dites fragiles dans des photobioréacteurs comportant des pompes centrifuges pour faire circuler la culture. Ces microalgues fragiles semblent d'autant plus sensibles aux sollicitations mécaniques qu'elles forment des chaînes et/ou qu'elles présentent des appendices tels que soies, flagelles, et spicules. Certaines microalgues, comme par exemple les algues du type Haematococcus pluvialis, perdent leur flagelles et s'enkystent en formant une paroi cellulaire épaisse et résistante. Par contre, d'autres microalgues, comme par exemple les algues du type Chlorella vulgaris ou Nannochloropsis oculata, ne présentent pas d'appendice et possèdent une paroi cellulaire épaisse, de sorte que celles-ci résistent au passage dans les moyens de pompage, et notamment dans les pompes centrifuges.

Il est cependant difficile d'identifier la nature des sollicitations mécaniques influençant la survie et la croissance des microorganismes. La plupart des auteurs s'accordent pour dire que les cisaillements et les accélérations ont le plus d'influence. Les cisaillements créent des tensions pouvant altérer l'intégrité cellulaire avec déchirement de la paroi des microorganismes et épanchement du cytosol. Les accélérations altèrent la structure de la cellule par augmentation du champ gravitationnel.

Les cellules vivantes sont mal préparées à ces efforts, et peut-être plus encore les cellules aquatiques qui vivent en équilibre hydrostatique et qui n'ont pas développé de structure capable de vaincre un champ gravitationnel. De surcroît, les cellules aquatiques sont sensibles à des valeurs seuils et probablement aussi aux variations et à la durée d'exposition. Dans l'état actuel des connaissances, il est difficile de prédire les effets mécaniques des conditions hydrodynamiques imposées aux cellules.

L'un des objets de la présente invention est de réduire les effets mécaniques imposées aux microorganismes, notamment les effets du type cisaillement et accélération, afin d'étendre le nombre d'espèces cultivables à l'intérieur du réacteur à celles qui sont les plus sensibles à ces effets mécaniques dommageables, autrement dit d'offrir un réacteur permettant la culture des microorganismes fragiles, comme par exemple les microalgues fragiles citées ci-dessus.

En outre, la demanderesse a observé que le rendement en culture des photobioréacteurs équipés de gazosiphon ou de pompe centrifuge était limité du fait notamment de la formation de bulles. En effet, la demanderesse a établi que le rendement en culture dépend en partie des phénomènes impliqués dans le transfert gaz-liquide pour éviter les pertes et réduire ce poste de dépense important. La modélisation du transfert gaz-liquide du dioxyde de carbone destiné à la réaction et de l'oxygène qu'elle produit nécessite la détermination de la vitesse de transfert qui est fonction du coefficient de transfert surfacique.

Le coefficient de transfert surfacique est un paramètre clé qui traduit les performances d'un système d'échange gaz/liquide à l'état stable. Ce coefficient de transfert surfacique est égale au produit du coefficient volumique de transfert de matière vers le liquide « KL » (m.s$^{-1}$) et de l'aire interfaciale rapportée au volume « a » (m$^{-1}$), où :

$$a = (\alpha_G.S)/V$$

a : Aire interfaciale rapportée au volume (m$^{-1}$) ;
$\alpha_G$ : coefficient de rétention de phase ;
S : Surface de contact (m$^2$) ; et
V : Volume du réacteur (m$^3$).

Le coefficient de transfert surfacique dépend donc de la géométrie du système d'échange gaz/liquide mais aussi des propriétés physicochimiques du liquide et du gaz. Dans le cas d'un échange gaz/liquide au sein d'une colonne à bulles verticale, la surface d'échange dépend du nombre de bulles et de leur taille. La population de bulles générée par une injection de gaz dans un liquide dépend du débit d'injection, de la géométrie de l'injecteur, et de la différence de pression de part et d'autre de celui-ci.

La présente invention a notamment pour but de fournir un procédé qui permette la culture de masse des microorganismes photosynthétiques, et son extension aux espèces les plus fragiles, avec un réacteur qui réponde aux problématiques suivantes :

- réduire voire éviter les sollicitations mécaniques liées en général à l'agitation et à la mise en circulation du milieu de culture et qui diminuent les performances de survie et de croissance des microorganismes photosynthétiques tels que les microalgues, et plus particulièrement les microalgues en chaînes pourvues d'appendices ;
- réduire voire éviter la production de bulles de petites dimensions susceptibles de favoriser l'agrégation des molécules organiques et le développement des microorganismes hétérotrophes à qui elles servent de substrat ;
- tout en réalisant le transfert photonique, pour délivrer le rayonnement solaire aux microorganismes photosynthétiques, le transfert de masse ou transfert gaz/liquide indispensable pour apporter le carbone et évacuer l'oxygène, et le transfert thermique, pour évacuer les calories apportées par le rayonnement et maintenir la culture à la bonne température ; et
- tout en maintenant des conditions mécaniques préservant l'intégrité des cellules et évitant les échanges avec le milieu environnant de nature à se prêter aux contaminations et aux disséminations.

A cet effet, le procédé utilise un réacteur photosynthétique adapté pour la culture de microorganismes photosynthétiques, notamment d'algues, comprenant :

- au moins une canalisation de réaction photosynthétique dans laquelle circule le milieu de culture liquide et qui est pourvue d'au moins un tronçon de réaction sensiblement horizontal et réalisé au moins partiellement dans un matériau transparent au rayonnement lumineux, ladite canalisation présentant une extrémité basse disposée en partie basse du réacteur et une extrémité haute disposée en partie haute du réacteur au-dessus de l'extrémité basse ;
- au moins une canalisation de retour assurant la liaison fluidique entre l'extrémité basse et l'extrémité haute de la canalisation de réaction ;
- au moins un moyen de mise en circulation du milieu de culture liquide ;
- au moins un moyen d'injection de gaz disposé dans le tronçon de réaction ou en amont dudit tronçon de réaction par rapport au sens de circulation du gaz, ledit moyen d'injection de gaz permettant d'injecter du gaz dans le réacteur ; et
- au moins un moyen d'échappement disposé en partie haute du réacteur et permettant l'échappement du gaz injecté dans le réacteur ;

dans lequel la disposition du moyen d'injection de gaz et/ou la conformation de la canalisation de réaction ou de la canalisation de retour sont conçues pour que le gaz injecté par le moyen d'injection remonte jusqu'au moyen d'échappement en circulant dans la canalisation de réaction, dans un sens de circulation allant de l'extrémité basse jusqu'à l'extrémité haute de la canalisation de réaction, de sorte que le gaz injecté et le milieu de culture liquide établissent un écoulement diphasique gaz/liquide dans le tronçon de réaction sensiblement horizontal.

Avec ce réacteur le milieu de culture liquide et le gaz circulent simultanément au contact l'un de l'autre le long du tronçon de réaction sensiblement horizontal et transparent, et échangent certains composants le long de leur parcours commun. Le gaz injecté en point bas s'échappe en point haut du réacteur tandis que le liquide circule selon une boucle sous l'impulsion d'un ou plusieurs moyens de mise en circulation. Les échanges sont proportionnels à la longueur du ou des tronçons transparents, si bien que l'effet de celle-ci est réduit, ce qui permet d'envisager de grands accroissements d'échelle.

Le réacteur est ainsi spécialement conçu pour accroître l'efficacité du transfert gaz-liquide et pour diminuer les sollicitations mécaniques infligées aux organismes en culture afin d'étendre la production aux espèces fragiles. En outre, le réacteur permet de limiter la formation de bulles de petit diamètre et ainsi de réduire le développement des microorganismes hétérotrophes consommateurs d'oxygène. En effet, avec le réacteur le transfert gaz/liquide ne se fait plus à l'intérieur d'une colonne à bulles verticale mais le long d'un tronçon de canalisation sensiblement horizontal dans lequel l'écoulement suit un régime du type diphasique horizontal.

Ainsi, le réacteur permet d'obtenir des échanges gaz-liquide au travers d'une interface formée au niveau de la surface libre du liquide dans la canalisation de réaction et plus particulièrement dans le ou les tronçons de réaction sensiblement horizontaux dans lesquels la circulation du gaz et du liquide s'établit sensiblement horizontalement selon un régime d'écoulement diphasique gaz/liquide du type écoulement stratifié ou écoulement à poches ou à bulles allongées.

Contrairement au principe mentionné ci-dessus selon lequel la réaction n'a lieu que dans la phase liquide, la demanderesse est partie du principe selon lequel le gaz fait partie intégrante de la réaction et doit être admis dans le volume réactionnel au même titre que le liquide. En privilégiant des régimes d'écoulement diphasiques horizontaux (stratifié, à poches ou à bulles allongées), la surface d'échange entre le gaz et le liquide est étendue à la totalité du parcours dans la canalisation de réaction (autrement dit le long de chaque tronçon de réaction sensiblement horizontal) avec une production de bulles nettement moins abondantes que dans le cas des réacteurs de l'art antérieur, réduisant de ce fait l'effet délétère observé pour ces bulles.

En outre, dans le réacteur la mise en circulation du milieu de culture liquide est assurée par un ou plusieurs moyens de mise en circulation générant des forces de cisaillement et centrifuges réduites : la fonction de mise en circulation étant dissociée de celle d'échange gaz-liquide contrairement au cas des réacteurs avec gazosiphon.

Selon une caractéristique, le réacteur comprend au moins un moyen d'injection de liquide permettant d'injecter du liquide dans le réacteur, le moyen d'échappement étant conformé pour permettre l'échappement du volume de liquide en excédent dans le réacteur en même temps que l'échappement du gaz injecté. Ceci permet ainsi de renouveler le milieu liquide et d'assurer un échappement de volume liquide excédentaire, en sus du volume gazeux injecté.

Dans une première réalisation, le moyen de mise en circulation est disposé dans la canalisation de retour pour mettre en circulation le milieu de culture liquide dans la canalisation de réaction de l'extrémité basse jusqu'à l'extrémité haute de ladite canalisation de réaction, dans le même sens de circulation que le gaz injecté. Dans cette réalisation, le gaz et le liquide circulent dans le même sens à l'intérieur de la canalisation de réaction et on parle d'un mode de circulation à co-courant. Avec le mode co-courant, les fluides (gaz et liquide) vont dans le même sens et leur contact dure quelques secondes avant d'être séparés du fait des différences de vitesse. La circulation à co-courant du gaz et du liquide crée une surface d'échange proportionnelle à la longueur de la canalisation de réaction, ce qui rend possible les accroissements de la taille de celle-ci sans avoir à multiplier le nombre d'appareils de dégazage et de carbonatation.

Dans une seconde réalisation, le moyen de mise en circulation est disposé dans la canalisation de retour pour mettre en circulation le milieu de culture liquide dans la canalisation de réaction de l'extrémité haute jusqu'à l'extrémité basse de ladite canalisation de réaction, dans un sens de circulation opposé au sens de circulation du gaz injecté. Dans cette réalisation, le gaz et le liquide circulent dans des sens opposés à l'intérieur de la canalisation de réaction et on parle d'un mode de circulation à contre-courant. La demanderesse a ainsi observé que la circulation à contre-courant est plus performante que la circulation à co-courant, bien que le recours au mode contre-courant pose des problèmes de régulation car, avec le mode contre-courant, les fluides se séparent immédiatement pour ne se retrouver à nouveau en contact que bien plus tard.

Selon une caractéristique, le moyen d'injection de gaz est disposé en partie basse du réacteur, entre le moyen de mise en circulation et l'extrémité basse de la canalisation de réaction. Dans cette configuration, le moyen de mise en circulation a tendance à évacuer le gaz en direction de la canalisation de réaction, évitant ainsi l'accumulation de gaz dans la canalisation de retour.

Selon une autre caractéristique, la canalisation de retour présente un dénivelé situé entre le moyen de mise en circulation et le moyen d'injection de gaz, ledit dénivelé formant une différence de niveaux entre le moyen de mise en circulation et le moyen d'injection de gaz pour éviter que le gaz injecté par le moyen d'injection ne se déplace en direction du moyen de mise en circulation.

Dans un mode de réalisation particulier, le moyen de mise en circulation est un moyen de propulsion mécanique disposé dans la canalisation de retour.

De façon préférentielle, le moyen de mise en circulation comprend une hélice entraînée en rotation par un moteur, et la canalisation de retour présente un logement de section élargie à l'intérieur duquel ladite hélice est mobile en rotation.

Avantageusement, le logement de l'hélice est disposé entre une zone de divergence et une zone de convergence de la circulation du milieu de culture liquide, afin d'assurer, dans la canalisation de retour, une continuité hydraulique sans variation brusque des vitesses dans le but de limiter les pertes de charge, les accélérations et les efforts de cisaillement subis par les microorganismes.

Selon une caractéristique avantageuse, la canalisation de retour présente en partie haute une zone élargie, et le moyen d'échappement est disposé dans ladite zone élargie de la canalisation de retour, permettant ainsi de diminuer la vitesse du liquide dans la zone élargie où s'effectue l'échappement du gaz et ainsi éviter des entraînements de gaz vers le bas par le liquide.

Selon une autre caractéristique, le moyen d'échappement est disposé en amont du moyen de mise en circulation du milieu de culture liquide par rapport au sens de circulation du gaz, afin d'éviter que le gaz ne circule à travers ledit moyen de mise en circulation et ne nuise à son fonctionnement.

Dans une réalisation préférée, le réacteur comprend au moins un corps de nettoyage conformé pour circuler à l'intérieur des canalisations de réaction et de retour et pour passer à travers le moyen de mise en circulation du milieu de culture liquide. Le ou les corps de nettoyage permettent ainsi de nettoyer l'intérieur du réacteur.

Avantageusement, le corps de nettoyage est conformé pour laisser passer au moins partiellement le gaz circulant à l'intérieur de la canalisation de réaction tout en étant adapté pour être entraîné par la circulation du milieu de culture liquide, afin que le corps n'influe pas sur la différence de vitesses entre le gaz et le milieu liquide ; ladite différence de vitesses influant directement sur les transferts de masse entre la phase liquide et la masse gazeuse.

Encore avantageusement, le corps de nettoyage est réalisé sous la forme d'une brosse comprenant un assemblage de poils, crins, brins ou équivalents, ou sous la forme d'une sphère creuse en matière élastomère dont au moins une partie de la surface est percée de trous. Ainsi, les poils émergés ou les trous laissent passer le gaz tandis que la partie immergée du corps de nettoyage constitue un obstacle à la circulation du milieu liquide de sorte à pouvoir être entraîné avec ce milieu liquide.

Dans un mode de réalisation particulier, le réacteur comprend :

- une canalisation de court-circuit disposée en parallèle de la canalisation de retour et reliant deux points de connexion prévus sur le réacteur, dont un premier point de connexion disposé sur la canalisation de réaction et un deuxième point de connexion disposé sur la canalisation de retour ou sur la canalisation de réaction ;
- deux vannes disposées de part et d'autre dudit premier point de connexion avec l'une des vannes disposée sur la canalisation de court-circuit ; et
- deux vannes disposées de part et d'autre dudit deuxième point de connexion avec l'une des vannes disposée sur la canalisation de court-circuit ; de sorte que la manipulation des vannes permet d'isoler la portion du réacteur située entre le premier et le deuxième points de connexion du côté de la canalisation de réaction et que le mélange gaz/milieu de culture liquide circule dans la canalisation de court-circuit et dans la portion du réacteur non isolée située entre le premier et le deuxième points de connexion du côté de la canalisation de retour.

Dans ce mode de réalisation, le réacteur est muni d'une canalisation de court-circuit qui permet l'isolement d'un sous-volume de la canalisation de réaction. Il est ainsi possible, dans un premier temps, que ce sous-volume soit isolé, inoculé et mis en culture. Puis, lorsque la concentration atteint un niveau suffisant dans ce sous-volume, les quatre vannes sont basculées dans l'état opposé de telle sorte que le reste du réacteur soit mis en circulation et inoculé par le sous-volume. Le procédé de l'invention envisage également un ensemble de réacteurs photosynthétiques adaptés pour la culture de microorganismes photosynthétiques comprenant au moins deux réacteurs conformes à l'invention, à savoir un premier et un deuxième réacteurs, et comprenant au moins une canalisation de liaison assurant une liaison fluidique entre le premier réacteur et le deuxième réacteur et au moins une vanne disposée sur ladite canalisation de liaison.

Cet ensemble est particulièrement avantageux pour avoir deux ou plus réacteurs en parallèle qui sont connectables, notamment pour permettre une inoculation, via la canalisation de liaison, afin de réaliser un ensemble de production qui soit productif cohérent. Afin de rendre possible l'inoculation d'un réacteur par son voisin dont la concentration aura atteint un stade avancé, il est possible d'interconnecter ces deux réacteurs de telle sorte que leurs contenus soient mélangés.

Selon une caractéristique, l'ensemble comprend deux canalisations de liaison entre les deux réacteurs, chacune pourvue d'une vanne, dont :

- une première canalisation de liaison reliant un point d'entrée disposé sur le premier réacteur en amont du moyen de mise en circulation dudit premier réacteur à un point de sortie disposé sur le deuxième réacteur en aval du moyen de mise en circulation dudit deuxième réacteur ; et
- une deuxième canalisation de liaison reliant un point d'entrée disposé sur le deuxième réacteur en amont du moyen de mise en circulation dudit deuxième réacteur à un point de sortie disposé sur le premier réacteur en aval du moyen de mise en circulation dudit premier réacteur.

Pour procéder à l'inoculation du deuxième réacteur à partir du premier réacteur déjà en service et dont la concentration a atteint le niveau d'exploitation, le deuxième réacteur à inoculer est rempli de milieu nutritif stérile et la circulation est établie en ouvrant les deux vannes des canalisations de liaison pour établir un échange croisé entre les deux réacteurs. L'interconnexion entre les deux réacteurs est établie entre l'amont et l'aval des moyens de mise en circulation pour que la force propulsive obtenue avec ces moyens de mise en circulation favorise la circulation d'échange.

Selon une autre caractéristique, l'ensemble comprend une pompe interposée sur la ou l'une des canalisations de liaison pour réduire la durée des échanges entre les deux réacteurs.

L'invention porte sur un procédé de culture de microorganismes photosynthétiques, notamment d'algues, utilisant le réacteur mentionné ci-dessus et comprenant les étapes suivantes :

- injection d'un milieu de culture liquide dans le réacteur selon un débit contrôlé ;
- injection d'un gaz dans le réacteur selon un débit contrôlé avec le moyen d'injection de gaz ;
- mise en circulation du milieu de culture liquide avec le moyen de mise en circulation ;
- contrôle du moyen de mise en circulation et du moyen d'injection de gaz pour établir dans le tronçon de réaction un régime d'écoulement diphasique gaz/liquide du type écoulement stratifié ou écoulement à poches ou à bulles allongées.

Comme décrit ci-dessus, l'établissement d'un régime d'écoulement diphasique gaz/liquide sensiblement horizontal, du type écoulement stratifié ou écoulement à poches ou à bulles allongées, présente de nombreux avantages comme la réduction de la production de bulles et l'augmentation du rendement du réacteur en augmentant notamment la surface d'échange entre le gaz et le liquide.

Selon une caractéristique, l'étape de contrôle comprend une étape de contrôle de la vitesse de circulation du liquide dans la canalisation de réaction entre environ 0,1 et 0,2 m/s afin d'établir un régime d'écoulement diphasique du type écoulement stratifié.

Selon une autre caractéristique, l'étape de contrôle comprend une étape de contrôle de la vitesse de circulation du liquide dans la canalisation de réaction entre environ 0,2 et 1 m/s afin d'établir un régime d'écoulement diphasique du type écoulement à poches ou à bulles allongées.

De façon avantageuse, l'étape de contrôle comprend une étape de contrôle de la vitesse de circulation du gaz entre environ 0,5 et 0,8 m/s, correspondant à un régime de vitesses adéquat pour les débits nécessaires à la réaction.

De façon encore avantageuse, le moyen de mise en circulation comprend une hélice entraînée en rotation par un moteur et dans lequel la vitesse de rotation de l'hélice est inférieure à environ 100 tours par minute, afin de limiter les sollicitations mécaniques au sein du milieu de culture liquide.

D'autres caractéristiques et avantages de la présente invention apparaîtront à la lecture de la description détaillée ci-après, d'un exemple de mise en oeuvre non limitatif, faite en référence aux figures annexées dans lesquelles :

- la figure 1 est une vue schématique de face d'un réacteur photosynthétique;
- la figure 2 est une vue schématique de côté du réacteur illustré à la figure 1 ;
- la figure 3 est une vue schématique partielle de l'intérieur du réacteur illustré à la figure 1 ;
- la figure 4 est une vue schématique de face d'un autre réacteur photosynthétique;
- la figure 5 est une vue schématique partielle de deux réacteurs et reliées entre eux via des canalisations de liaison ;
- les figures 6a et 6b illustrent respectivement l'évolution de l'oxygène dissous et du dioxyde de carbone dissous en fonction du temps pour un premier réacteur;
- les figures 7a et 7b illustrent respectivement l'évolution de l'oxygène dissous et du dioxyde de carbone dissous en fonction du temps pour un second réacteur;
- les figures 8a à 8ef illustrent de façon schématique plusieurs régimes d'écoulement dans une conduite horizontale, respectivement un écoulement à bulles dispersées, un écoulement à bulles allongées, un écoulement stratifié lisse, un écoulement stratifié ondulé, un écoulement à poches, et un écoulement annulaire.

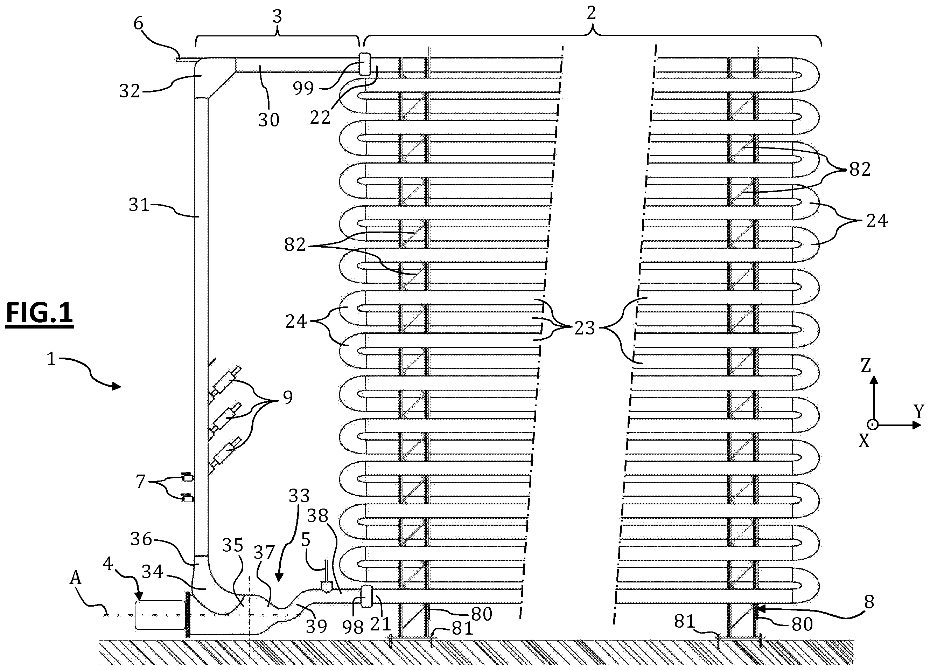

Les figures 1 à 3 illustrent un réacteur 1 photosynthétique et qui est adapté pour la culture de microorganismes photosynthétiques, notamment d'algues, et en particulier pour la culture de microorganismes photosynthétiques fragiles aux sollicitations mécaniques et donc inadaptés pour la culture dans des réacteurs de l'état de la technique.

Le réacteur 1 comporte :

- au moins une canalisation de réaction 2 photosynthétique dans laquelle circule le milieu de culture liquide, ladite canalisation de réaction 2 présentant une extrémité basse 21 disposée en partie basse du réacteur 1 et une extrémité haute 22 disposée en partie haute du réacteur 1 au-dessus de l'extrémité basse 21 ;
- au moins une canalisation de retour 3 assurant la liaison fluidique entre l'extrémité basse 21 et l'extrémité haute 22 de la canalisation de réaction 2 ;
- au moins un moyen de mise en circulation 4 du milieu de culture liquide ;
- au moins un moyen d'injection de gaz 5 permettant d'injecter du gaz dans le réacteur ; et
- au moins un moyen d'échappement 6 disposé en partie haute du réacteur 1 et permettant l'échappement du gaz injecté dans le réacteur 1 ;
- au moins un moyen d'injection de liquide 7 permettant d'injecter du liquide dans le réacteur 1.

Il est bien entendu au sens de la présente demande que l'emploi des termes « basse » et « haute » pour désigner des parties du réacteur 1, ainsi que l'emploi d'un terme comme « au-dessus de », font référence à la disposition relative des éléments ou parties du réacteur selon la direction verticale illustrée par la flèche Z sur les figures.

Comme visible en figure 1, la canalisation de réaction 2 comprend plusieurs tronçons horizontaux 23, dits tronçons de réaction, reliés successivement par des tronçons coudés 24. Les tronçons horizontaux 23 et les tronçons coudés 24 sont connectés en série à des intervalles pour que la canalisation de réaction 2 présente une forme de conduite continue en accordéon ou en spirale entre son extrémité basse 21 et son extrémité haute 22. Cette canalisation de réaction 2 s'étend principalement selon un plan vertical, avec les tronçons horizontaux 23 disposés successivement les uns au-dessus des autres.

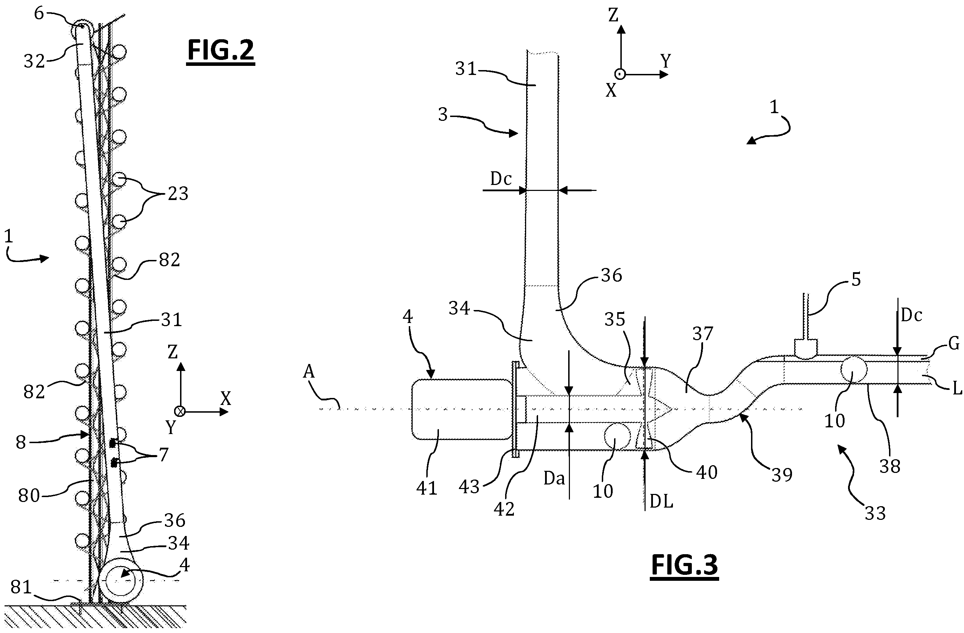

Comme visible en figure 2, les tronçons horizontaux 23 sont disposés successivement de part et d'autre d'une structure de support 8 verticale comprenant :

- des piliers verticaux 80 fixés dans le sol via des vis 81 ; et
- des traverses 82 solidaires des piliers verticaux 81 et supportant les tronçons horizontaux 23.

La structure de support 8 reprend le poids de la canalisation de réaction 2 avec ses deux piliers verticaux 81 qui sont espacés l'un de l'autre d'une distance n'excédant pas la limite de portée des tronçons horizontaux 23 provoquant la rupture desdits tronçons horizontaux 23 remplis de liquide et de gaz du fait de leur poids, et des traverses 82 qui maintiennent les tronçons horizontaux 23 à équidistance verticale et horizontale les uns des autres.

Les tronçons horizontaux 23 sont réalisés au moins partiellement dans un matériau transparent au rayonnement solaire, comme par exemple dans un matériau en verre ou en matériau synthétique type plastique ou polymère acrylique comme le plexiglas™. Ces tronçons horizontaux 23 sont réalisés sous la forme de tuyaux rectilignes et de section circulaire de diamètre Dc. Les tronçons coudés 24 sont réalisés sous la forme de tuyaux coudés à 180° pour relier bout à bout deux tronçons horizontaux 23 successifs. Les tronçons coudés 24 sont de section circulaire de diamètre Dc tout comme les tronçons horizontaux.

La canalisation de retour 3 présente une forme générale de « C » et comporte successivement :

- un tronçon supérieur 30 horizontal relié à l'extrémité haute 22 de la canalisation de réaction 2 via un raccord 99 ;
- un tronçon central 31 vertical ou légèrement oblique, comme visible en figure 2, relié au tronçon supérieur 30 par un premier coude 32 formant un angle d'environ 90° ; et
- un tronçon inférieur 33 relié d'une part au tronçon central 31 par un deuxième coude 34 formant un angle d'environ 90° et d'autre part à l'extrémité basse 21 de la canalisation de réaction 2 via un raccord 98, et venant dans le prolongement du tronçon horizontal 23 le plus bas de la canalisation de réaction 2 dit le premier tronçon horizontal.

La canalisation de retour 3 est réalisée dans une matière non transparente au rayonnement solaire et/ou peut être disposée à l'abri de la lumière à l'intérieur d'un local fermé, tandis que la canalisation de réaction 2 est exposée à la lumière solaire, éventuellement sous une serre.

Le tronçon supérieur 30 est réalisé sous la forme d'un tuyau rectiligne et de section circulaire de diamètre Dc égal à celui des tronçons horizontaux 23 de la canalisation de réaction 2. Le tronçon supérieur 30 vient dans le prolongement du tronçon horizontal 23 situé le plus haut de la canalisation de réaction 2, dit le dernier tronçon horizontal, et s'étend au même niveau vertical que ce dernier tronçon horizontal 23.

Le tronçon central 31 est également réalisé sous la forme d'un tuyau rectiligne et de section circulaire de diamètre Dc égal à celui des tronçons horizontaux 23 de la canalisation de réaction 2. Ce tronçon central 31 s'étend selon une direction verticale ou légèrement oblique par rapport à la direction verticale Z.

Le premier coude 32, disposé en partie haute, présente une section élargie par rapport au tronçon supérieur 30 et au tronçon central 31 de même diamètre Dc. Le moyen d'échappement 6 est disposé sur ledit premier coude 32 et se présente sous la forme d'un tuyau de dimensions prédéterminées qui débouche à l'intérieur du premier coude 32. Ainsi, la section de passage au niveau du premier coude 32 est augmentée pour diminuer la vitesse du liquide dans ledit premier coude 32 et éviter des entraînements de gaz vers le bas dans le tronçon central 31. Le moyen d'échappement 6 est disposé en partie haute par rapport au tronçon horizontal 23 le plus haut de la canalisation de réaction 2, de telle sorte que seul l'excès de liquide s'échappe du réacteur 1 en même temps que le gaz. Le moyen d'échappement 6 est calibré pour permettre l'échappement du volume de liquide en excédent dans le réacteur 1 en même temps que l'échappement du gaz injecté.

Le réacteur 1 comprend deux moyens d'injection de liquide 7 disposés sur le tronçon central 31, à savoir un premier et un second moyens d'injection permettant d'injecter respectivement le milieu de culture liquide et l'inoculum dans le réacteur 1. Ces moyens d'injection 7 se présentent sous la forme de ports d'injection permettant une connexion à une source avec contrôle de l'asepsie.

Le réacteur 1 comprend en outre un ou plusieurs capteurs 9 disposés sur la canalisation de retour 3 et plus particulièrement sur le tronçon central 31, et adaptés pour fournir les signaux nécessaires au contrôle de la réaction, notamment des signaux représentatifs de la température, du pH, du taux d'oxygène dissous et de la la turbidité du milieu liquide. Ce contrôle sert notamment à réguler les injections de gaz et de liquide dans le réacteur 1.

Le tronçon inférieur 33 présente un logement 35, de section élargie par rapport au diamètre Dc du tronçon central 31, destiné à recevoir en partie le moyen de mise en circulation 4. Ce logement 35 est situé dans le prolongement direct du deuxième coude 35 et s'étend selon une direction principale A horizontale.

Le moyen de mise en circulation 4 illustré aux figures 1 à 3 est disposé dans la canalisation de retour 3, au moins en partie à l'intérieur du logement 35, pour mettre en circulation le milieu de culture liquide dans la canalisation de réaction 2 de l'extrémité basse 21 jusqu'à l'extrémité haute 22 de ladite canalisation de réaction 2, autrement dit de bas en haut à l'intérieur de la canalisation de réaction 2. Dans cette configuration, le milieu liquide circule du tronçon supérieur 30 en direction du tronçon inférieur 33 à l'intérieur de la canalisation de retour 3, autrement dit de haut en bas à l'intérieur de la canalisation de retour 3 et de son tronçon central 31.

Le moyen de mise en circulation 4 est réalisé sous la forme d'un moyen de propulsion mécanique qui comprend une hélice 40 entraînée en rotation par un moteur rotatif 41 via un arbre de sortie 42 dudit moteur 41. Le moteur 41 est disposé à l'extérieur du réacteur 1, au niveau du deuxième coude 34, et est fixé sur une plaque 43 fixée de façon amovible sur les parois de la canalisation de retour 3, notamment au moyen de vis de fixation. L'arbre de sortie 42 traverse de façon étanche ladite plaque 43, débouche à l'intérieur du logement 35, et supporte l'hélice 40 qui est ainsi mobile en rotation à l'intérieur de ce logement 35. L'arbre de sortie 42 et l'hélice 40 tournent autour d'un axe de rotation A horizontal et l'hélice 40 s'étend dans un plan sensiblement vertical.

L'emplacement du moyen de mise en circulation 4 en partie basse de la canalisation de retour 3 permet un accès facile pour l'entretien. Dans l'exemple illustré aux figures 1 à 3, il est en effet aisé d'entretenir l'hélice 40 par démontage de la plaque 43 fermant le logement 35 d'accueil de l'hélice 40. L'ensemble mécanique que constituent le moteur 41, la plaque 43, l'arbre de sortie 42 et l'hélice 40 peut être dégagé simplement par translation horizontale, par exemple en prenant appui sur le sol.

Le tronçon inférieur 33 présente une partie rectiligne 38 horizontal réalisée sous la forme d'un tuyau rectiligne et de section circulaire de diamètre Dc égal à celui des tronçons horizontaux 23 de la canalisation de réaction 2. La partie rectiligne 38 est disposée entre le logement 35 et l'extrémité basse 21 de la canalisation de réaction 2. La partie rectiligne 38 vient dans le prolongement du premier tronçon horizontal 23 (celui situé le plus bas de la canalisation de réaction 2), et s'étend au même niveau vertical que ce premier tronçon horizontal 23.

Une continuité hydraulique sans variation brusque des vitesses est recherchée dans la canalisation de retour 3 pour limiter les pertes de charge, les accélérations et les efforts de cisaillement subis par les microorganismes photosynthétiques. La vitesse moyenne du milieu liquide diminue entre le tronçon central 31 et le tronçon inférieur 33 du fait de l'augmentation de la section de passage entre le tronçon central 31 et le logement 35, puis augmente du fait de la diminution inverse de la section de passage entre le logement 35 et la partie rectiligne 38. Afin d'assurer la continuité hydraulique entre le tronçon central 31 et la partie rectiligne 38 du tronçon inférieur 33, un divergent 36 est disposé en partie haute du deuxième coude 34, autrement dit en amont du logement 35 et de l'hélice 40, et un convergent 37 est disposé entre le logement 35 et la partie rectiligne 38, autrement dit en aval du logement 35 et de l'hélice 40.

Le tronçon inférieur 33 présente un dénivelé 39 situé entre le logement 35 et la partie rectiligne 38, et plus particulièrement entre le convergent 37 et la partie rectiligne 38. Ce dénivelé 39 est réalisé sous la forme de deux coudes formant une différence de niveaux entre le logement 35 et la partie rectiligne 38.

Le moyen d'injection de gaz 5 est disposé sur la partie rectiligne 38 du tronçon inférieur 33 de la canalisation de retour 3, de sorte que le gaz G injecté par le moyen d'injection de gaz 5 remonte jusqu'au moyen d'échappement 6 en circulant dans la canalisation de réaction 2, dans un sens de circulation allant de l'extrémité basse 21 jusqu'à l'extrémité haute 22 de la canalisation de réaction 2, autrement dit de bas en haut à l'intérieur de la canalisation de réaction 2. Dans cette configuration, le gaz G et le milieu liquide L circule à co-courant, c'est-à-dire dans le même sens de circulation, à l'intérieur de la canalisation de réaction 2.

Le dénivelé 39 est disposé en amont du moyen d'injection de gaz 5 pour éviter que le gaz injecté par le moyen d'injection de gaz 5 ne se déplace dans le mauvais sens, c'est-à-dire en direction du logement 35 et de l'hélice 40, en particulier lorsque le moteur 41 est à l'arrêt.

L'emplacement du moyen de mise en circulation 4, et de l'hélice 40 en particulier, en partie basse du tronçon central 31 de la canalisation de retour 3 et en amont du moyen d'injection de gaz 5 est avantageux pour permettre une évacuation par entraînement horizontal du gaz injecté dans la partie rectiligne 38 du tronçon inférieur 33 de la canalisation de retour 3.

La disposition du moyen d'échappement 6 en amont du moyen de mise en circulation 4 conjugué à la disposition du moyen de mise en circulation 4 en amont du moyen d'injection de gaz 5 est avantageuse pour éviter que le gaz ne circule à travers l'hélice 40 et ne nuise à son fonctionnement. En effet, la présence de gaz gêne le fonctionnement de la plupart des moyens de propulsion mécanique et aux hélices en particulier, et son accumulation doit donc être évitée au risque de faire caviter l'hélice 40.

Comme visible en figure 3, le gaz G injecté par le moyen d'injection de gaz 5 et le milieu de culture liquide L mis en circulation au moyen de l'hélice 40 établissent ensemble un écoulement diphasique gaz/liquide dans la partie rectiligne 38 et donc nécessairement dans le tronçon horizontal 23 le plus bas de la canalisation de réaction 2. Le gaz G, comme visible en figure 3, forme un ciel à l'intérieur de la partie rectiligne 38 et donc du tronçon horizontal 23 le plus bas de la canalisation de réaction 2 ; ce ciel se formant également dans les tronçons horizontaux 23 suivants au fur et à mesure de l'avancée du gaz dans la canalisation de réaction 2 jusqu'au moyen d'échappement 6.

En fonction notamment des vitesses respectives de circulation du gaz et du milieu liquide, l'écoulement diphasique gaz/liquide suivra un régime d'écoulement diphasique du type écoulement stratifié (ciel de gaz continu) ou du type écoulement à poches ou à bulles allongées (ciel de gaz discontinu). Ces régimes d'écoulement sont possibles car la circulation de gaz, à co-courant ou à contre-courant de la circulation du milieu liquide, se fait principalement dans la partie rectiligne 38 horizontale du tronçon inférieur 33, dans les tronçons horizontaux 23 et dans le tronçon supérieur 30 horizontal. Bien entendu, le gaz monte dans la canalisation de réaction 2 via les tronçons coudés 24, mais ces remontées de gaz dans les tronçons coudés 24 ne nuisent pas au régime d'écoulement stratifié, à poches ou à bulles allongées observé dans les tronçons horizontaux 23.

De manière générale, le moyen d'injection de gaz 5 est disposé en amont du premier tronçon horizontal 23 (celui le plus bas dans la canalisation de réaction 2) ou éventuellement dans ledit premier tronçon horizontal 23 par rapport au sens de circulation du gaz de bas en haut à l'intérieur de la canalisation de réaction 2. Il est également envisageable de prévoir plusieurs moyens d'injection de gaz en différents points de la canalisation de réaction 2, sur un ou plusieurs tronçons horizontaux 23, et éventuellement d'autres moyens d'échappement du gaz.

Concernant les écoulements diphasiques dans des conduites horizontales, des travaux ont mis en évidence plusieurs régimes d'écoulement selon les conditions de vitesse, de diamètre, de température, de nature, de pression des fluides circulant, à savoir notamment :

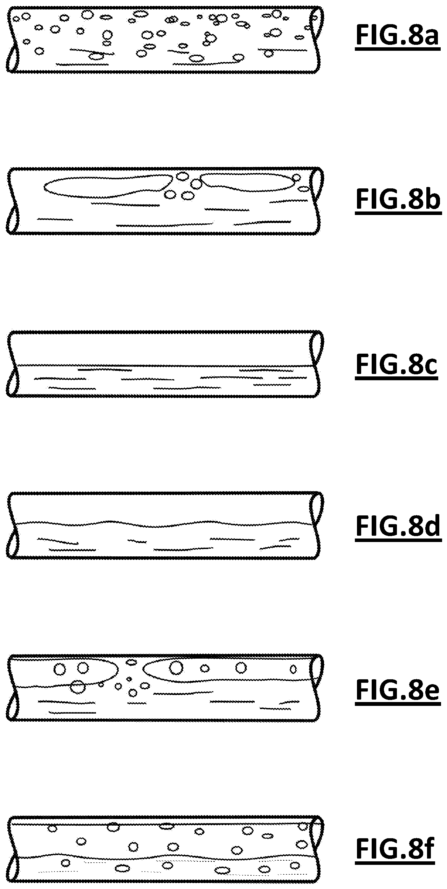

- écoulement à bulles dispersées, dispersed bubbles flow, de typologie de Mandhane AD, qui est illustré en figure 8a ; et
- écoulement à bulles allongées, ou elongated bubbles flow, de typologie de Mandhane I, qui est illustré en figure 8b ;
- écoulement stratifié, ou stratified flow, qui est illustré en figure 8c avec un écoulement stratifié ondulé et en figure 8d avec un écoulement stratifié lisse, de typologie de Mandhane SS et SW, respectivement;
- écoulement à poches, ou slug flow, de typologie de Mandhane I, qui est illustré en figure 8e ;
- écoulement annulaire, ou annular mist flow, de typologie de Mandhane AD, qui est illustré en figure 8f.

Dans le cas de la présente invention, les régimes d'écoulement choisis se situent au niveau de la transition SS/I dans la typologie de Mandhane, c'est-à-dire entre le régime stratifié et le régime à poches ou à bulles allongées. Dans le régime stratifié, l'interface gaz/liquide est constituée par la surface libre, dont la largeur varie avec le niveau du liquide dans les canalisations. Dans le régime à poches ou à bulles allongées, l'interface gaz/liquide est constituée par le plancher et le plafond de la poche ou de la bulle allongée.

Dans le cas de la présente invention, avec un diamètre Dc pour les tronçons horizontaux 23 de l'ordre de quelques centimètres, par exemple compris entre environ 4 et environ 15 centimètre, l'écoulement diphasique gaz/liquide dans les tronçons horizontaux 23 suivra un régime d'écoulement stratifié pour des vitesses du liquide comprises entre 0,1 et 0,2 m/s, et un régime d'écoulement à poches ou à bulles allongées pour des vitesses du liquide comprises entre 0,2 et 1 m/s.

Des vitesses de liquide supérieures à 0,2 m/s pourront être choisies afin de favoriser le mélange dans le milieu de culture liquide, impliquant ainsi que l'écoulement diphasique dans les tronçons horizontaux 23 suivra un régime d'écoulement à poches ou à bulles allongées.

Les échanges ou transferts gaz/liquide variant sensiblement de façon proportionnelle à la différence de vitesse entre la circulation du gaz et la circulation du milieu liquide, il est particulièrement avantageux de maintenir élevée la différence de vitesse entre la circulation gazeuse et la circulation liquide. La demanderesse a ainsi observé que la vitesse du gaz devrait être stabilisée entre 0,5 et 0,8 m/s pour les débits gazeux nécessaires à la réaction photosynthétique, et que la différence de vitesse nécessaire aux échanges est aisée à obtenir avec une circulation à co-courant.

La circulation à co-courant du gaz et du liquide crée une surface d'échange proportionnelle à la longueur de la canalisation de réaction 2, ce qui rend possible les accroissements de la longueur de ladite canalisation de réaction 2 sans avoir à multiplier le nombre d'appareils de dégazage et de carbonatation. Bien que cette introduction de gaz dans la canalisation de réaction implique une diminution du volume réactionnel liquide, allant jusqu'à 15% selon le débit gazeux, à l'intérieur de la canalisation de réaction 2, cette diminution du volume réactionnel liquide est en grande partie compensée par l'accroissement de la productivité volumique qui résulte de la diminution de la longueur du chemin optique. Du point de vue de la qualité microbiologique, l'absence des petites bulles dans le réacteur 1 conforme à l'invention présente un grand intérêt, ainsi que décrit ci-dessus, et ce réacteur 1 présente également un intérêt en terme de coûts car il ne nécessite pas d'appareil de dégazage et de carbonatation.

La demanderesse a également observé qu'il est possible d'accroître la différence de vitesse entre la circulation gazeuse et la circulation liquide, et donc d'accroître les transferts gaz/liquide, en appliquant une circulation à contre-courant au sein de la canalisation de réaction 2, autrement dit en faisant en sorte que le milieu liquide circule de haut en bas à l'intérieur de la canalisation de réaction 2. Ainsi, dans une réalisation non illustrée, le moyen de mise en circulation 4 est disposé dans la canalisation de retour 3 pour mettre en circulation le milieu de culture liquide dans la canalisation de réaction 2 de l'extrémité haute 22 jusqu'à l'extrémité basse 21 de ladite canalisation de réaction 2, dans un sens de circulation opposé au sens de circulation du gaz injecté, autrement dit de haut en bas à l'intérieur de la canalisation de réaction 2. Dans cette variante, le milieu liquide circule du tronçon inférieur 33 en direction du tronçon supérieur 30 à l'intérieur de la canalisation de retour 3, autrement dit de bas en haut à l'intérieur de la canalisation de retour 3 et de son tronçon central 31. Pour réaliser une telle circulation à contre-courant, le moyen de mise en circulation 4 peut être retourné à 180° pour fonctionner dans l'autre sens de circulation au même emplacement que dans le cas précédent di à co-courant, puisqu'il a l'avantage d'être exempt de circulation gazeuse, en partie à l'intérieur du même logement 35 adéquat. Les moyens d'injection de gaz 5 et d'échappement 6 peuvent être avantageusement maintenus aux mêmes emplacements que dans le cas précédent, puisqu'ils maximisent la longueur du parcours commun et donc la durée du contact fluidique.

Le tableau suivant compare les performances de trois réacteurs photosynthétiques implantés sous le 42ème parallèle et consistant dans une canalisation de réaction transparente de 215 m de long et de 76 mm de diamètre intérieur, avec une contenance globale de 1000 litres.

|  | Réacteur à « colonne à bulles » | Réacteur selon l'invention « à co-courant » | Réacteur selon l'invention « à contre-courant » |
|---|---|---|---|
| S = Surface de contact (m$^2$) | 2 | 7 | 7 |
| KL.a = Coefficient de transfert surfacique (heure$^{-1}$) | 1 | 3 | 4 |
| VM = Vitesse maximum d'oxygénation du milieu liquide (mg/L/heure) | 44 | 44 | 44 |
| VE = Vitesse d'extraction d'O2 (mg/L/h) | 11 | 47 | 58 |
| Efficacité = VE/VM (pourcentage) | 25 | 107 | 132 |

Le réacteur à « colonne à bulles » correspond au réacteur de l'état de la technique décrit ci-dessus et comportant un gazosiphon. Ce réacteur à « colonne à bulles » comprend une colonne à bulle de hauteur égale à 4 m et de diamètre égal à 76 mm identique à celui de la canalisation de réaction.

Le réacteur « à co-courant » correspond au réacteur dans lequel un mode de circulation à co-courant est établi dans la canalisation de réaction, tandis que le réacteur « à contre-courant » correspond au réacteur dans lequel un mode de circulation à contre-courant est établi dans la canalisation de réaction.

La vitesse VM maximale d'oxygénation a été calculée pour une espèce d'algues à croissance rapide au plus fort de l'activité photosynthétique, c'est-à-dire en période estivale et à midi.

La demanderesse confirme ainsi que l'extraction d'oxygène par le réacteur connu à « colonne à bulles » est insuffisante et limite l'efficacité du réacteur, alors que les réacteurs conformes à l'invention, « à co-courant » et « à contre-courant », couvrent efficacement les besoins d'extraction d'oxygène.

Le transfert gaz/liquide et donc l'extraction d'oxygène est plus efficace avec les réacteurs selon l'invention, où l'interface gaz/liquide se fait sur la totalité du parcours de la canalisation de réaction, qu'avec le réacteur connu « à colonne à bulles », où l'interface gaz/liquide est limitée aux petites bulles et où l'extraction d'oxygène est localisée. Ceci confirme l'opinion de la demanderesse mentionnée ci-dessus selon laquelle le gaz fait partie intégrante de la réaction et doit être admis dans le volume réactionnel au même titre que le liquide.

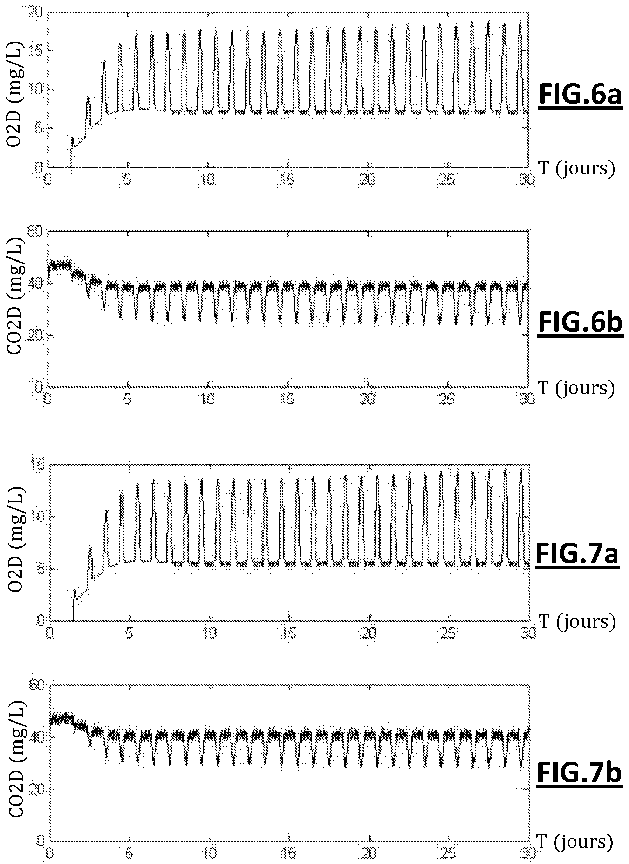

En outre, le mode de circulation à contre-courant est plus performant que le mode de circulation à co-courant. Les figures 6 et 7 confirment ce point en illustrant des simulations numériques des échanges gazeux dans le cas d'une culture de microalgues du type Porphyridium cruentum dans un réacteur recevant un débit de gaz contenant 3% de $CO_2$, et 0% d'$O_2$ injecté de 6 litres par minute avec un mode de circulation à co-courant (figures 6a et 6b) et à contre courant (figures 7a et 7b), où :

- la figure 6a illustre l'évolution de l'oxygène dissous O2D (en mg/L) en fonction du temps (en jours) pour un réacteur à co-courant ;
- la figure 6b illustre l'évolution du dioxyde de carbone dissous CO2D (en mg/L) en fonction du temps (en jours) pour un réacteur à co-courant ;
- la figure 7a illustre l'évolution de l'oxygène dissous O2D (en mg/L) en fonction du temps (en jours) pour un réacteur à contre courant ; et

- la figure 7b illustre l'évolution du dioxyde de carbone dissous CO2D (en mg/L) en fonction du temps (en jours) pour un réacteur à contre courant.

Ces simulations numériques confirment donc l'efficacité supérieure du mode à contre-courant. Le mode de circulation à co-courant demeure tout de même suffisant tout le temps pour maintenir la teneur en oxygène en-dessous de 20 mg/L, autrement dit le mode à co-courant est suffisant si le seuil de toxicité se situe à 20 mg/L.

Cependant, le recours au mode à contre-courant pose des problèmes de régulation. En effet, avec le mode à co-courant, les fluides (gaz et liquide) circulent dans le même sens et leur contact est réalisé pendant quelques secondes avant d'être séparés l'un de l'autre du fait des différences de vitesse. Au contraire, avec le mode à contre-courant, les fluides se séparent immédiatement pour ne se retrouver à nouveau en contact que bien plus tard.

De manière générale, il est possible de jouer sur le débit gazeux et sur la vitesse de circulation du milieu de culture liquide, ainsi qu'aux variations d'intensité lumineuse, pour les adapter aux différentes espèces cultivées : un fort débit gazeux est par exemple souhaitable par forte intensité lumineuse et lorsque l'espèce algale présente une croissance rapide afin d'accroître les échanges gaz-liquide. Toutefois, il faut tenir compte du fait qu'une variation du débit gazeux entraîne celle du volume de culture. Ainsi, l'augmentation du débit gazeux provoque une diminution du volume liquide pouvant conduire à un dénoyage du dernier tronçon horizontal 23 (celui situé le plus haut) et à l'interruption de la circulation dans la canalisation de circulation 2.

Comme visible en figure 3, le réacteur 1 peut également comprendre un ou plusieurs corps de nettoyage 10 conformés pour circuler à l'intérieur des canalisations de réaction 2 et de retour 3 afin de nettoyer l'intérieur de ces canalisations 2 et 3. Pour pouvoir circuler en boucle dans le réacteur 1, le ou les corps de nettoyage 10 sont également conformés pour passer à travers le ou les moyens de mise en circulation 4 du milieu de culture liquide, autrement dit à travers les pales de l'hélice 40 dans le mode de réalisation particulier décrit ci-dessus.

Les corps de nettoyage 10, de préférence sphérique, présentent un diamètre sensiblement égal au diamètre Dc intérieur de la canalisation de réaction 2 et de ses tronçons horizontaux 23 et coudés 24 ; le nettoyage du réacteur 1 concernant essentiellement la paroi interne de la canalisation de réaction 2 où a lieu la culture des microorganismes. Les corps de nettoyage 10 sont de préférence en matière souple afin d'absorber par déformation les sollicitations mécaniques du moyen de mise en circulation 4, par exemple les impacts des pales de l'hélice 40 lors de la traversée du logement 35.

Le logement 35 est avantageusement de section circulaire, du moins dans le plan de rotation de l'hélice 40, c'est-à-dire le plan vertical au droit de ladite hélice 40. Comme visible en figure 3, cette section circulaire du logement 35 est de diamètre DL légèrement supérieur au diamètre de l'hélice 40. Ce diamètre DL est également supérieur au diamètre Dc de la canalisation de réaction 2 et du tronçon central 31 de la canalisation de retour 3 afin que l'hélice 40 puisse livrer passage aux corps de nettoyage 10. Comme indiqué ci-dessus, cette augmentation du diamètre se traduit par une diminution des vitesses linéaires du fluide et des vitesses de rotation de l'hélice. Dans ce cas, le diamètre DL du logement 35 diminué du diamètre Da de l'arbre de sortie 42 du moteur 41 doit être au moins le double du diamètre Dc de la canalisation de réaction 2 pour que les corps de nettoyage 10 puissent circuler librement, entraînés par le milieu réactionnel liquide, y compris à travers l'hélice 40, soit :

$$DL - Da = 2.Dc.$$

Dans le mode de réalisation illustré à la figure 3, le diamètre DL est égale au triple du diamètre Dc intérieur de la canalisation de réaction 2 et le diamètre Da de l'arbre de sortie 42 est égal au diamètre Dc intérieur de la canalisation de réaction 2. Dans cette configuration, les corps de nettoyage 10 de diamètre Dc peuvent circuler dans la partie annulaire de largeur égale à Dc, s'étendant entre la paroi interne du logement 35 et l'arbre 42, et peuvent ainsi passer plus facilement entre les pales de l'hélice 40.

Afin de réduire l'obstacle que les pales de l'hélice 40 constituent pour ces corps de nettoyage 10 et pour les microorganismes, ces pales sont de préférence en nombre limité à un (formant ainsi une sorte de vis d'Archimède) ou à deux. Afin de diminuer la surface de balayage des pales et les effets mécaniques qui en résultent, la vitesse de rotation de l'hélice 40 sera faible et de préférence inférieure à 100 tours par minute.

La circulation des corps de nettoyage 10 dans les tronçons horizontaux 23 peut avoir pour effet de rendre les vitesses de circulation du gaz et du milieu liquide sensiblement identiques dans ces tronçons horizontaux 23 puisqu'ils constituent des obstacles pour l'un et l'autre fluide, et les contraignent à progresser à la même vitesse aux débits de fuite près.

Cependant, comme décrit ci-dessus, la différence de vitesses entre la circulation gazeuse et la circulation liquide influe directement sur les transferts de masse gaz/liquide et doit avantageusement être maintenue au niveau le plus élevé possible. C'est la raison pour laquelle le corps de nettoyage ne doit pas empêcher le passage du gaz. Pour cela, le corps de nettoyage est conformé pour laisser passer au moins partiellement le gaz circulant à l'intérieur de la canalisation de réaction 2 tout en étant adapté pour être entraîné par la circulation du milieu de culture liquide. A cet effet, le ou chaque corps de nettoyage 10 est réalisé sous la forme d'une brosse sphérique comprenant un assemblage de poils, crins, brins ou équivalents, avec une partie centrale porteuse de ces poils. Ainsi, dans les tronçons horizontaux 23, les poils émergés laissent passer le gaz au niveau du ciel de gaz et la partie centrale immergée et porteuse des poils présente un diamètre suffisamment grand pour constituer un obstacle au passage du liquide, de sorte que le milieu liquide entraine avec lui le corps de nettoyage 10.

De la même manière, le corps de nettoyage 10 peut être réalisé sous la forme d'une sphère creuse en matière élastomère dont une partie substantielle de la surface est percée de trous qui permettent de laisser passer le gaz.

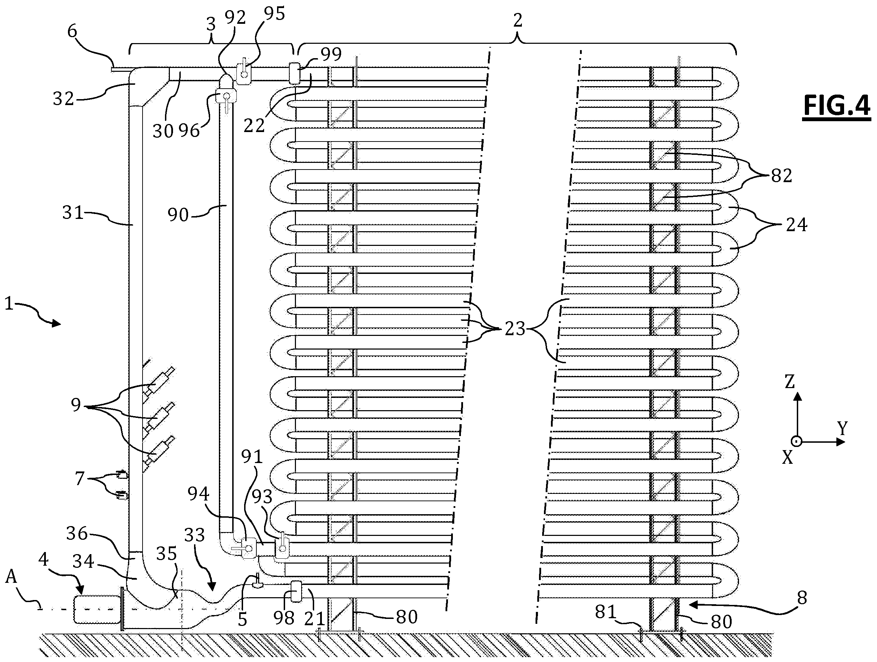

Une première amélioration du réacteur 1 illustrée en figure 4 consiste à équiper le réacteur 1 décrit ci-dessus de :

- une canalisation de court-circuit 90 disposée en parallèle de la canalisation de retour 3 et reliant deux points de connexion prévus sur le réacteur 1, dont un premier point de connexion 91 disposé sur la canalisation de réaction 2 et un deuxième point de connexion 92 disposé sur la canalisation de retour 3 ou sur la canalisation de réaction 2 (le premier point de connexion 91 étant bien entendu distinct du deuxième point de connexion) ;
- deux vannes 93, 94 disposées de part et d'autre dudit premier point de connexion 91 avec l'une des vannes 94 disposée sur la canalisation de court-circuit 90 ; et
- deux vannes 95, 96 disposées de part et d'autre dudit deuxième point de connexion avec l'une des vannes 96 disposée sur la canalisation de court-circuit 90.

Dans le mode de réalisation illustré en figure 4 :

- le premier point de connexion 91 est disposé sur un tronçon coudé 34 ou sur un tronçon horizontal 23 situé au-dessus du premier tronçon horizontal (celui situé le plus bas) et en-dessous du dernier tronçon horizontal (celui situé le plus haut) ; et
- le deuxième point de connexion 92 est disposé sur la canalisation de retour 3, entre l'extrémité haute 22 de la canalisation de réaction 2 et le moyen d'échappement 6 pour permettre l'échappement du gaz dans la boucle de court-circuit, et en particulier sur le tronçon supérieur 30 ;
- la vanne 93 est disposée en amont du premier point de connexion 91 par rapport au sens de circulation du gaz, juste après ce premier point de connexion 91 ;
- la vanne 94 est disposée sur la canalisation de court-circuit 90, juste après le premier point de connexion 91 ;
- la vanne 95 est disposée entre le deuxième point de connexion 92 et l'extrémité haute 22 de la canalisation de réaction 2, en aval du deuxième point de connexion 92 par rapport au sens de circulation du gaz ; et
- la vanne 96 est disposée sur la canalisation de court-circuit 90, juste avant le deuxième point de connexion 92.

La manipulation des vannes 93 à 96, qui sont par exemple du type vanne d'isolement à passage intégral, permet d'isoler la portion du réacteur 1 située entre les deux points de connexion 91, 92 du côté de la canalisation de réaction 2, et permet que le mélange gaz/milieu de culture liquide circule dans une boucle de court-circuit comprenant la portion du réacteur 1 non isolée située entre les deux points de connexion 91, 92 du côté de la canalisation de retour 3 et la canalisation de court-circuit 90. La canalisation de court-circuit 90 est réalisée sous la forme d'un tuyau rectiligne, vertical et de section circulaire de diamètre Dc égal à celui des tronçons horizontaux 23 de la canalisation de réaction 2.

Dans le mode de réalisation illustré en figure 4, les vannes 93 et 95 sont fermées tandis que les vannes 94 et 96 sont ouvertes de sorte que le mélange circule dans la boucle de retour.

Ce réacteur amélioré, muni d'un court-circuit, permet ainsi de n'exploiter qu'un sous-volume de réaction, correspondant à la portion de la canalisation de réaction 2 disposée dans la boucle de court-circuit, et qui peut représenter environ 1/10 du volume total de la canalisation de réaction 2. Sur la figure 4, ce sous-volume réactionnel correspond au volume entre l'extrémité basse 21 et le premier point de connexion 91 qui correspond à deux tronçons horizontaux 23, à comparer aux vingt six tronçons horizontaux de la canalisation de réaction 2 donnée à titre d'exemple.

La boucle de court-circuit, ou boucle réduite, possède toutes les fonctionnalités de la grande boucle (circuit complet comme dans le cas du réacteur de la figure 1) puisqu'elle comporte :

- le tronçon central 31 avec le ou les moyens d'injection de liquides 7 et le ou les capteurs 9 ;
- le moyen de mise en circulation 4 ;
- le moyen d'injection de gaz 5 ;
- le moyen d'échappement 6 ; et
- un sous-volume de réaction qui comprend au moins un tronçon horizontal 23.

L'utilisation d'un tel réacteur peut se faire de la manière suivante : après que la totalité du réacteur 1 ait été remplie avec du milieu stérile, les quatre vannes 92 à 96 sont manipulées pour obtenir la configuration illustrée en figure 4. Dans un premier temps, le sous-volume réactionnel est inoculé et mis en culture. Dans un deuxième temps, lorsque la concentration en microorganismes atteint un niveau suffisant dans le sous-volume réactionnel, les quatre vannes 92 à 96 sont basculées dans l'état opposé de telle sorte que le reste de la canalisation de réaction 2 soit mis en circulation et inoculé par le sous-volume réactionnel. On parle alors d'un réacteur à inoculation par intraconnexion.

Dans une variante non illustrée de réalisation du réacteur à inoculation par intraconnexion :

- le premier point de connexion 91 est disposé sur la canalisation de réaction 2, sur un tronçon coudé 34 ou un tronçon horizontal 23 situé au-dessus du premier tronçon horizontal et en-dessous du dernier tronçon horizontal ; et
- le deuxième point de connexion 92 est disposé également sur la canalisation de réaction 2, au-dessus du premier point de connexion 91 (afin de court-circuiter la partie de la canalisation de retour disposée entre les deux points de connexion).

Dans une autre variante non illustrée de réalisation du réacteur à inoculation par intraconnexion :

- le premier point de connexion 91 est disposé sur la canalisation de réaction 2, sur un tronçon coudé 34 ou un tronçon horizontal 23 situé au-dessus du premier tronçon horizontal et en-dessous du dernier tronçon horizontal ; et
- le deuxième point de connexion 92 est disposé sur la canalisation de retour 3, entre le moyen d'injection de gaz 5 et l'extrémité basse 21 de la canalisation de réaction 2 (afin de court-circuiter la partie de la canalisation de retour disposée entre son extrémité basse et le premier point de connexion).

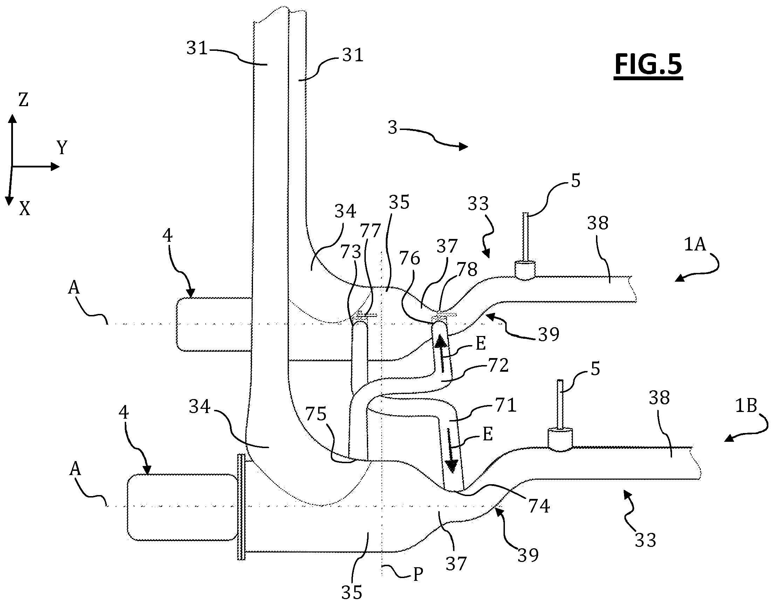

Comme illustré en figure 5, l'invention concerne également un ensemble de réacteurs photosynthétiques comprenant au moins deux réacteurs 1A, 1B à savoir un premier 1A et un deuxième 1B réacteurs, et comprenant au moins une canalisation de liaison 71, 72 assurant une liaison fluidique entre le premier réacteur 1A et le deuxième réacteur 1B et au moins une vanne 77, 78 disposée sur ladite canalisation de liaison 71, 72, afin de permettre l'inoculation d'un réacteur par l'autre réacteur.

Dans le mode de réalisation illustré en figure 5, l'ensemble comprend deux canalisations de liaison 71, 72 entre les deux réacteurs 1A, 1B, chacune pourvue de deux vannes respectivement 77, 78 au niveau du premier réacteur 1A, et de deux autres vannes (non visibles et formant les vis-à-vis des deux vannes 77, 78) au niveau du deuxième réacteur 1B, dont :

- une première canalisation de liaison 71 reliant un point d'entrée 73 disposé sur le premier réacteur 1A dans le logement 35 en amont de l'hélice (non visible) dudit premier réacteur 1A à un point de sortie 74 disposé sur le deuxième réacteur 1B en aval de l'hélice (également non visible) dudit deuxième réacteur 1B ; et
- une deuxième canalisation de liaison 72 reliant un point d'entrée 75 disposé sur le deuxième réacteur 1B dans le logement 35 en amont de l'hélice (non visible) dudit deuxième réacteur 1B à un point de sortie 76 disposé sur le premier réacteur 1A en aval de l'hélice (également non visible) dudit premier réacteur 1A.

Les réacteurs 1A, 1B sont assemblés de façon parallèle pour constituer un ensemble productif cohérent. Afin de rendre possible l'inoculation d'un réacteur par son voisin dont la concentration en microorganismes aurait atteint un stade avancé, l'ensemble prévoit d'interconnecter ces deux réacteurs avec les canalisations de liaison 71, 72 de telle sorte que leurs contenus respectifs soient mélangés.

Dans le mode de réalisation illustré sur la figure 5, les plans de rotation des deux hélices sont confondus dans un seul et un même plan P vertical.

En outre, comme visible en figure 5, chaque point de sortie 74, 76 est placé à l'extrémité du convergent 37 correspondant pour bénéficier d'un effet Venturi ; lesdits convergents 37 étant disposés entre les logements 35 respectifs et les parties rectilignes 38 respectives.

En outre, chaque point d'entrée 73, 75 est placé dans le logement 35 correspondant de l'hélice, de préférence en amont du plan de rotation P correspondant.

Chaque canalisation de liaison 71, 72 comprend au moins une vanne 77, 78 permettant la connexion sous asepsie des deux canalisations de liaison 71, 72 qui relient de façon croisée et symétrique les points d'entrée et les points de sortie des deux réacteurs 1A, 1B à interconnecter. Chaque canalisation de liaison 71, 72 peut également comprendre deux vannes, une à son point d'entrée et une à son point de sortie.

L'utilisation d'un tel ensemble peut se faire de la manière suivante : pour procéder à l'inoculation du deuxième réacteur 1B à partir du premier réacteur 1A déjà en service et dont la concentration en microorganismes a atteint le niveau d'exploitation, les vannes 77, 78 et leurs vis-à-vis sont fermées dans un premier temps et le deuxième réacteur 1B à inoculer est rempli de milieu nutritif stérile, et ensuite la circulation est établie à l'intérieur du deuxième réacteur 1B. Dans un deuxième temps, les vannes 77, 78 et leurs vis à vis sont ouvertes et un échange croisé s'établit entre les deux réacteurs comme illustré par les flèches E de la figure 5.

Après l'ouverure des vannes 77, 78 et de leurs vis à vis, les concentrations deviennent sensiblement égales dans les deux réacteurs 1A et 1B et il est possible de les isoler par fermeture des vannes 77, 78 et de leurs vis-à-vis. Pour réduire la durée de cet échange, une pompe (non visible) peut être interposée sur l'une et/ou l'autre des canalisations de liaison 71, 72.

Dans le cas où la mise en circulation est obtenue dans les réacteurs par d'autres moyens propulsifs que l'hélice, et que les réacteurs ne présentent pas de variation de diamètre formant un convergent, l'effet Venturi ne peut être obtenu. Dans une telle configuration non illustrée, l'interconnexion entre les deux réacteurs est établie entre l'amont et l'aval des deux moyens de mise en circulation, et la force propulsive établit la circulation d'échange dans l'autre sens que celui indiqué sur la figure 5.

Bien entendu l'exemple de mise en oeuvre évoqué ci-dessus ne présente aucun caractère limitatif et d'autres détails et améliorations peuvent être apportés au réacteur selon l'invention, sans pour autant sortir du cadre de l'invention où d'autres formes de canalisation de réaction et/ou de canalisation de retour et/ou de moyens de mise en circulation peuvent par exemple être réalisées. Ainsi, les tronçons horizontaux 23 peuvent présenter une légère inclinaison par rapport à l'horizontalité, par exemple de quelques degrés autour de la direction horizontale.

**Revendications**

1. Procédé de culture de microorganismes photosynthétiques, notamment d'algues, utilisant un réacteur (1) photosynthétique comprenant :

   - au moins une canalisation de réaction (2) photosynthétique dans laquelle circule le milieu de culture liquide et qui est pourvue d'au moins un tronçon de réaction (23) sensiblement horizontal et réalisé au moins partiellement dans un matériau transparent au rayonnement lumineux, ladite canalisation de réaction (2) présentant une extrémité basse (21) disposée en partie basse du réacteur (1) et une extrémité haute (22) disposée en partie haute du réacteur (1) au-dessus de l'extrémité basse (21) ;
   - au moins une canalisation de retour (3) assurant la liaison fluidique entre l'extrémité basse (21) et l'extrémité haute (22) de la canalisation de réaction (2) ;
   - au moins un moyen de mise en circulation (4) du milieu de culture liquide ;
   - au moins un moyen d'injection de gaz (5) disposé dans le tronçon de réaction (23) ou en amont dudit tronçon de réaction (23) par rapport au sens de circulation du gaz, ledit moyen d'injection de gaz (5) permettant d'injecter du gaz dans le réacteur (1) ;
   - au moins un moyen d'injection de liquide (7) permettant d'injecter du liquide dans le réacteur (1) ; et
   - au moins un moyen d'échappement (6) disposé en partie haute du réacteur (1) et permettant l'échappement du gaz injecté dans le réacteur (1) ;

   ledit procédé comprenant les étapes suivantes :

   - injection d'un milieu de culture liquide dans le réacteur (1) selon un débit contrôlé avec le moyen d'injection de liquide (7) ;
   - injection d'un gaz dans le réacteur (1) selon un débit contrôlé avec le moyen d'injection de gaz (5) ;
   - mise en circulation du milieu de culture liquide avec le moyen de mise en circulation (4) ;
   - contrôle du moyen de mise en circulation (4) et du moyen d'injection de gaz (5) pour établir dans le tronçon de réaction (23) un régime d'écoulement diphasique gaz/liquide horizontal du type écoulement stratifié ou écoulement à poches ou à bulles allongées, où le gaz injecté par le moyen d'injection (5) remonte jusqu'au moyen d'échappement (6) en circulant dans la canalisation de réaction (2), dans un sens de circulation allant de l'extrémité basse (21) jusqu'à l'extrémité haute (22) de la canalisation de réaction (2), de sorte que le gaz injecté et le milieu de culture liquide établissent ledit régime d'écoulement diphasique gaz/liquide horizontal dans le tronçon de réaction (23) sensiblement horizontal ;

   où la mise en circulation du milieu de culture liquide n'est pas réalisée par un gazosiphon ou dispositif d'ascenseur à gaz, de sorte que le transfert gaz/liquide ne se fait pas à l'intérieur d'une colonne à bulles mais le long d'un tronçon de réaction (23) sensiblement horizontal dans lequel l'écoulement suit ledit régime d'écoulement diphasique gaz/liquide horizontal.

2. Procédé selon la revendication 1 dans lequel le moyen de mise en circulation (4) est disposé dans la canalisation de retour (3) et met en circulation le milieu de culture liquide dans la canalisation de réaction (2) de l'extrémité basse (21) jusqu'à l'extrémité haute (22) de ladite canalisation de réaction (2), dans le même sens de circulation que le gaz injecté.

3.  Procédé selon la revendication 1, dans lequel le moyen de mise en circulation (4) est disposé dans la canalisation de retour (3) et met en circulation le milieu de culture liquide dans la canalisation de réaction (2) de l'extrémité haute (22) jusqu'à l'extrémité basse (21) de ladite canalisation de réaction (2), dans un sens de circulation opposé au sens de circulation du gaz injecté.

4.  Procédé selon l'une quelconque des revendications 2 ou 3, dans lequel l'injection de gaz est réalisée avec le moyen d'injection de gaz (5) disposé en partie basse du réacteur (1), entre le moyen de mise en circulation (4) et l'extrémité basse (21) de la canalisation de réaction (2).

5.  Procédé selon la revendication 4, dans lequel la canalisation de retour (3) présente un dénivelé (39) situé entre le moyen de mise en circulation (4) et le moyen d'injection de gaz (5), ledit dénivelé (39) formant une différence de niveaux entre le moyen de mise en circulation (4) et le moyen d'injection de gaz (5), évitant que le gaz injecté par le moyen d'injection de gaz (5) ne se déplace en direction du moyen de mise en circulation (4).

6.  Procédé selon l'une quelconque des revendications précédentes, dans lequel la mise en circulation du milieu de culture liquide est réalisée avec le moyen de mise en circulation (4) qui est un moyen de propulsion mécanique disposé dans la canalisation de retour (3).

7.  Procédé selon l'une quelconque des revendications précédentes, dans lequel le moyen d'échappement (6) est disposé en amont du moyen de mise en circulation (4) du milieu de culture liquide par rapport au sens de circulation du gaz, évitant que le gaz ne circule à travers ledit moyen de mise en circulation (4) et ne nuise à son fonctionnement.

8.  Procédé selon l'une quelconque des revendications précédentes, comprenant la circulation d'au moins un corps de nettoyage (10) à l'intérieur des canalisations de réaction (2) et de retour (3) et à travers le moyen de mise en circulation (4) du milieu de culture liquide.

9.  Procédé selon l'une quelconque des revendications précédentes, dans lequel le réacteur (1) comprend :

    - une canalisation de court-circuit (90) disposée en parallèle de la canalisation de retour (3) et reliant deux points de connexion (91, 92) prévus sur le réacteur (1), dont un premier point de connexion (91) disposé sur la canalisation de réaction (2) et un deuxième point de connexion (92) disposé sur la canalisation de retour (3) ou sur la canalisation de réaction (2) ;
    - deux vannes (93, 94) disposées de part et d'autre dudit premier point de connexion (91) avec l'une des vannes (94) disposée sur la canalisation de court-circuit (90) ; et
    - deux vannes (95, 96) disposées de part et d'autre dudit deuxième point de connexion (92) avec l'une des vannes (96) disposée sur la canalisation de court-circuit (90) ;

    et le procédé comprend une étape de manipulation des vannes (93, 94, 95, 96) pour isoler la portion du réacteur (1) située entre le premier (91) et le deuxième (92) points de connexion du côté de la canalisation de réaction (2) de sorte que le mélange gaz/milieu de culture liquide circule dans la canalisation de court-circuit (90) et dans la portion du réacteur (1) non isolée située entre le premier (91) et le deuxième (92) points de connexion du côté de la canalisation de retour (3).

10. Procédé selon l'une quelconque des revendications précédentes, dans lequel le procédé est mis en oeuvre dans un ensemble de réacteurs (1A; 1B) photosynthétiques adaptés pour la culture de microorganismes photosynthétiques comprenant au moins deux réacteurs (1A; 1B) conformes à l'une quelconque des revendications 1 à 12, à savoir un premier (1A) et un deuxième (1B) réacteurs, et comprenant au moins une canalisation de liaison (71, 72) assurant une liaison fluidique entre le premier réacteur (1A) et le deuxième réacteur (1B) et au moins une vanne (77, 78) disposée sur ladite canalisation de liaison (71, 72).

11. Procédé selon la revendication 10, dans lequel l'ensemble comprend deux canalisations de liaison (71, 72) entre les deux réacteurs (1A, 1B), chacune pourvue d'au moins une vanne (77, 78), dont :

    - une première canalisation de liaison (71) reliant un point d'entrée (73) disposé sur le premier réacteur (1A) en amont du moyen de mise en circulation (4) dudit premier réacteur à un point de sortie (74) disposé sur le deuxième réacteur (1B) en aval du moyen de mise en circulation (4) dudit deuxième réacteur (1B) ; et
- une deuxième canalisation de liaison (72) reliant un point d'entrée (75) disposé sur le deuxième réacteur (1B) en amont du moyen de mise en circulation (4) dudit deuxième réacteur (1B) à un point de sortie (76) disposé sur le premier réacteur (1A) en aval du moyen de mise en circulation (4) dudit premier réacteur (1A).

12. Procédé selon l'une quelconque des revendications 1 à 11, dans lequel l'étape de contrôle comprend une étape de contrôle de la vitesse de circulation du liquide dans la canalisation de réaction (2) entre 0,1 et 0,2 m/s afin d'établir un régime d'écoulement diphasique du type écoulement stratifié.

13. Procédé selon l'une quelconque des revendications 1 à 11, dans lequel l'étape de contrôle comprend une étape de contrôle de la vitesse de circulation du liquide dans la canalisation de réaction (2) entre 0,2 et 1 m/s afin d'établir un régime d'écoulement diphasique du type écoulement à poches ou à bulles allongées.

14. Procédé selon l'une quelconque des revendications précédentes, dans lequel l'étape de contrôle comprend une étape de contrôle de la vitesse de circulation du gaz entre 0,5 et 0,8 m/s.

15. Procédé selon l'une quelconque des revendications précédentes, dans lequel le moyen de mise en circulation (4) comprend une hélice (40) entraînée en rotation par un moteur (41) et dans lequel la vitesse de rotation de l'hélice est inférieure à 100 tours par minute.


**Patentansprüche**

1. Verfahren zur Kultivierung von photosynthetischen Mikroorganismen, insbesondere Algen, unter Verwendung eines Photosynthesereaktors (1), der Folgendes umfasst:

- mindestens eine Photosynthesereaktionsleitung (2), in der das flüssige Nährmedium zirkuliert und die mit mindestens einem im Wesentlichen waagerechten Reaktionsabschnitt (23) versehen ist und zumindest teilweise aus einem für Lichtstrahlung durchlässigen Material ausgeführt ist, wobei die Reaktionsleitung (2) ein unteres Ende (21) aufweist, das im unteren Teil des Reaktors (1) angeordnet ist, und ein oberes Ende (22), das im oberen Teil des Reaktors (1) über dem unteren Ende (21) angeordnet ist;
- mindestens eine Rückleitung (3), die für die Flüssigkeitsverbindung zwischen dem unteren Ende (21) und dem oberen Ende (22) der Reaktionsleitung (2) sorgt;
- mindestens ein Umwälzmittel (4) für das flüssige Nährmedium;
- mindestens ein Gaseinleitmittel (5), das in dem Reaktionsabschnitt (23) oder bezogen auf die Gasströmungsrichtung vor dem Reaktionsabschnitt (23) angeordnet ist, wobei das Gaseinleitmittel (5) das Einleiten von Gas in den Reaktor (1) ermöglicht;
- mindestens ein Flüssigkeitseinleitmittel (7), das das Einleiten von Flüssigkeit in den Reaktor (1) ermöglicht; und
- mindestens ein Ausströmmittel (6), das im oberen Teil des Reaktors (1) angeordnet ist und das Ausströmen des Gases ermöglicht, das in den Reaktor (1) eingeleitet wird;

wobei das Verfahren folgende Schritte umfasst:

- Einleiten eines flüssigen Nährmediums in den Reaktor (1) mit einem eingestellten Durchfluss mit dem Flüssigkeitseinleitmittel (7);
- Einleiten eines Gases in den Reaktor (1) mit einem eingestellten Durchfluss mit dem Gaseinleitmittel (5) ;
- Umwälzen des flüssigen Nährmediums mit dem Umwälzmittel (4);
- Einstellen des Umwälzmittels (4) und des Gaseinleitmittels (5) zum Herstellen eines waagerechten Gas-Flüssigkeit-Zweiphasenströmungsbereichs als geschichtete Strömung oder Schwallströmung oder Strömung mit länglichen Blasen in dem Reaktionsabschnitt (23), wo das von dem Einleitmittel (5) eingeleitete Gas bis zum Ausströmmittel (6) nach oben steigt und dabei in der Reaktionsleitung (2) in einer Strömungsrichtung von dem unteren Ende (21) bis zum oberen Ende (22) der Reaktionsleitung (2) strömt, sodass das eingeleitete Gas und das flüssige Nährmedium den waagerechten Gas-Flüssigkeit-Zweiphasenströmungsbereich in dem im Wesentlichen waagerechten Reaktionsabschnitt (23) herstellen;

wo das Umwälzen des flüssigen Nährmediums nicht über einen Gassiphon oder eine Gasliftvorrichtung erfolgt, sodass der Gas-Flüssigkeits-Transport nicht im Inneren einer Blasensäule erfolgt, sondern entlang eines im Wesentlichen waagerechten Reaktionsabschnitts (23), in dem die Strömung dem waagerechten Gas-Flüssigkeit-Zweiphasenströmungsbereich entspricht.

2. Verfahren nach Anspruch 1, wobei das Umwälzmittel (4) in der Rückleitung (3) angeordnet ist und das flüssige Nährmedium in der Reaktionsleitung (2) vom unteren Ende (21) bis zum oberen Ende (22) der Reaktionsleitung (2), in derselben Strömungsrichtung wie das eingeleitete Gas, umwälzt.

3. Verfahren nach Anspruch 1, wobei das Umwälzmittel (4) in der Rückleitung (3) angeordnet ist und das flüssige Nährmedium in der Reaktionsleitung (2) vom oberen Ende (22) bis zum unteren Ende (21) der Reaktionsleitung (2), in einer der Strömungsrichtung des eingeleiteten Gases entgegengesetzten Strömungsrichtung, umwälzt.

4. Verfahren nach Anspruch 2 oder 3, wobei die Gaseinleitung mit dem Gaseinleitmittel (5) erfolgt, das im unteren Teil des Reaktors (1), zwischen dem Umwälzmittel (4) und dem unteren Ende (21) der Reaktionsleitung (2), angeordnet ist.

5. Verfahren nach Anspruch 4, wobei die Rückleitung (3) eine Stufe (39) zwischen dem Umwälzmittel (4) und dem Gaseinleitmittel (5) aufweist, wobei sich durch die Stufe (39) ein Höhenunterschied zwischen dem Umwälzmittel (4) und dem Gaseinleitmittel (5) ergibt, wodurch verhindert wird, dass sich das von dem Gaseinleitmittel (5) eingeleitete Gas in Richtung des Umwälzmittels (4) bewegt.

6. Verfahren nach einem der vorhergehenden Ansprüche, wobei das Umwälzmittel für das flüssige Nährmedium mit dem Umwälzmittel (4) umgesetzt ist, das ein in der Rückleitung (3) angeordnetes mechanisches Antriebsmittel ist.

7. Verfahren nach einem der vorhergehenden Ansprüche, wobei das Ausströmmittel (6) bezogen auf die Gasströmungsrichtung vor dem Umwälzmittel (4) für das flüssige Nährmedium angeordnet ist, wodurch verhindert wird, dass das Gas durch das Umwälzmittel (4) strömt und seine Funktion beeinträchtigt.

8. Verfahren nach einem der vorhergehenden Ansprüche, das das Bewegen von mindestens einem Reinigungskörper (10) im Inneren der Reaktionsleitung (2) und der Rückleitung (3) und durch das Umwälzmittel (4) für das flüssige Nährmedium umfasst.

9. Verfahren nach einem der vorhergehenden Ansprüche, wobei der Reaktor (1) Folgendes umfasst:

   - eine Kurzschlussleitung (90), die parallel zur Rückleitung (3) angeordnet ist und zwei Anschlussstellen (91, 92) verbindet, die an dem Reaktor (1) vorgesehen sind, von denen eine erste Anschlussstelle (91) an der Reaktionsleitung (2) angeordnet ist und eine zweite Anschlussstelle (92) an der Rückleitung (3) oder an der Reaktionsleitung (2) angeordnet ist;
   - zwei Ventile (93, 94), die auf beiden Seiten der ersten Anschlussstelle (91) angeordnet sind, wobei eines der Ventile (94) an der Kurzschlussleitung (90) angeordnet ist; und
   - zwei Ventile (95, 96), die auf beiden Seiten der zweiten Anschlussstelle (92) angeordnet sind, wobei eines der Ventile (96) an der Kurzschlussleitung (90) angeordnet ist;

   und das Verfahren einen Schritt zum Betätigen der Ventile (93, 94, 95, 96) umfasst, damit der Abschnitt des Reaktors (1), der sich zwischen der ersten (91) und der zweiten (92) Anschlussstelle auf der Seite der Reaktionsleitung (2) befindet, abgesperrt wird, sodass die Mischung aus Gas und flüssigem Nährmedium in der Kurzschlussleitung (90) und in dem nicht abgesperrten Abschnitt des Reaktors (1) fließt, der sich zwischen der ersten (91) und der zweiten (92) Anschlussstelle auf der Seite der Rückleitung (3) befindet.

10. Verfahren nach einem der vorhergehenden Ansprüche, wobei das Verfahren in einer Anordnung aus Photosynthesereaktoren (1A; 1B) durchgeführt wird, die zur Kultivierung von photosynthetischen Mikroorganismen ausgelegt sind, die mindestens zwei Reaktoren (1A; 1B) nach einem der Ansprüche 1 bis 12 umfasst, das heißt einen ersten (1A) und einen zweiten (1B) Reaktor, und die mindestens eine Verbindungsleitung (71, 72) umfasst, die für eine Flüssigkeitsverbindung zwischen dem ersten Reaktor (1A) und dem zweiten Reaktor (1B) sorgt, und mindestens ein Ventil (77, 78), das an der Verbindungsleitung (71, 72) angeordnet ist.

11. Verfahren nach Anspruch 10, wobei die Anordnung zwei Verbindungsleitungen (71, 72) zwischen den beiden Reaktoren (1A, 1B) umfasst, die jeweils mit mindestens einem Ventil (77, 78) versehen sind, von denen:

- eine erste Verbindungsleitung (71) eine Einlaufstelle (73), die am ersten Reaktor (1A) in Strömungsrichtung vor dem Umwälzmittel (4) des ersten Reaktors angeordnet ist, mit einer Auslaufstelle (74) verbindet, die an dem zweiten Reaktor (1B) in Strömungsrichtung hinter dem Umwälzmittel (4) des zweiten Reaktors (1B) angeordnet ist; und
- eine zweite Verbindungsleitung (72) eine Einlaufstelle (75), die am zweiten Reaktor (1B) in Strömungsrichtung vor dem Umwälzmittel (4) des zweiten Reaktors (1B) angeordnet ist, mit einer Auslaufstelle (76) verbindet, die an dem ersten Reaktor (1A) in Strömungsrichtung hinter dem Umwälzmittel (4) des ersten Reaktors (1A) angeordnet ist.

12. Verfahren nach einem der Ansprüche 1 bis 11, wobei der Schritt des Einstellens einen Schritt des Einstellens der Strömungsgeschwindigkeit der Flüssigkeit in der Reaktionsleitung (2) zwischen 0,1 und 0,2 m/s umfasst, damit ein Zweiphasenströmungsbereich als geschichtete Strömung hergestellt wird.

13. Verfahren nach einem der Ansprüche 1 bis 11, wobei der Schritt des Einstellens einen Schritt des Einstellens der Strömungsgeschwindigkeit der Flüssigkeit in der Reaktionsleitung (2) zwischen 0,2 und 1 m/s umfasst, damit ein Zweiphasenströmungsbereich als Schwallströmung oder Strömung mit länglichen Blasen hergestellt wird.

14. Verfahren nach einem der vorhergehenden Ansprüche, wobei der Schritt des Einstellens einen Schritt des Einstellens der Gasströmungsgeschwindigkeit zwischen 0,5 und 0,8 m/s umfasst.

15. Verfahren nach einem der vorhergehenden Ansprüche, wobei das Umwälzmittel (4) einen Propeller (40) umfasst, der von einem Motor (41) in Drehung versetzt wird und wobei die Drehzahl des Propellers unter 100 Umdrehungen pro Minute beträgt.

**Claims**

1. Method for culturing photosynthetic microorganisms, in particular algae, using a photosynthetic reactor (1) comprising:

- at least one photosynthetic reaction pipe (2) in which the liquid culture medium circulates and which is provided with at least one substantially horizontal reaction section (23) that is at least partially made of a material that is transparent to light radiation, said reaction pipe (2) having a bottom end (21) placed in the bottom part of the reactor (1) and a top end (22) placed in the top part of the reactor (1) above the bottom end (21);
- at least one return pipe (3) which ensures the fluidic link between the bottom end (21) and the top end (22) of the reaction pipe (2);
- at least one means for circulating (4) the liquid culture medium;
- at least one gas injection means (5) placed in the reaction section (23) or upstream of said reaction section (23) relative to the direction of circulation of the gas, said gas injection means (5) making it possible to inject gas into the reactor (1) ;
- at least one liquid injection means (7) making it possible to inject liquid into the reactor (1); and
- at least one discharge means (6) arranged in the top part of the reactor (1) and allowing discharge of the gas injected into the reactor (1);

said method comprising the following steps:

- injecting a liquid culture medium into the reactor (1) according to a controlled flow rate with the liquid injection means (7);
- injecting a gas into the reactor (1) according to a controlled flow rate with the gas injection means (5);
- circulating the liquid culture medium with the circulating means (4);
- controlling the circulating means (4) and the gas injection means (5) so as to establish, in the reaction section (23), a horizontal gas/liquid two-phase flow regime of the stratified flow or pocket or bubble flow type, where the gas injected by the injection means (5) rises back up to the discharge means (6) by circulating in the reaction pipe (2), in a direction of circulation which goes from the bottom end (21) to the top end (22) of the reaction pipe (2), such that the gas injected and the liquid culture medium establish said horizontal gas/liquid two-phase flow regime in the substantially horizontal reaction section (23);

where the circulating of the liquid culture medium is not carried out by an air-lift or gas-lift device, such that the gas/liquid transfer does not take place inside a bubble column, but along a substantially horizontal reaction section (23) in which the flow follows said horizontal gas/liquid two-phase flow regime.

2. Method according to Claim 1, in which the circulating means (4) is placed in the return pipe (3) and circulates the liquid culture medium in the reaction pipe (2) from the bottom end (21) to the top end (22) of said reaction pipe (2), in the same direction of circulation as the injected gas.

3. Method according to Claim 1, in which the circulating means (4) is placed in the return pipe (3) and circulates the liquid culture medium in the reaction pipe (2) from the top end (22) to the bottom end (21) of said reaction pipe (2), in a direction of circulation which is opposite to the direction of circulation of the injected gas.

4. Method according to either one of Claims 2 and 3, in which the gas injection is carried out with the gas injection means (5) placed in the bottom part of the reactor (1), between the circulating means (4) and the bottom end (21) of the reaction pipe (2) .

5. Method according to Claim 4, in which the return pipe (3) has a height difference (39) located between the circulating means (4) and the gas injection means (5), said height difference (39) forming a difference in levels between the circulating means (4) and the gas injection means (5), preventing the gas injected by the gas injection means (5) from moving in the direction of the circulating means (4).

6. Method according to any one of the preceding claims, in which the circulating of the liquid culture medium is carried out with the circulating means (4) which is a mechanical propulsion means placed in the return pipe (3).

7. Method according to any one of the preceding claims, in which the discharge means (6) is placed upstream of the means for circulating (4) the liquid culture medium relative to the direction of circulation of the gas, preventing the gas from circulating through said circulating means (4) and from harming the operation thereof.

8. Method according to any one of the preceding claims, comprising the circulating of at least one cleaning body (10) inside the reaction pipe (2) and return pipe (3) and through the means for circulating (4) the liquid culture medium.

9. Method according to any one of the preceding claims, in which the reactor (1) comprises:

- a bypass pipe (90) placed in parallel with the return pipe (3) and connecting two connection points (91, 92) provided on the reactor (1), with a first connection point (91) being placed on the reaction pipe (2) and a second connection point (92) being placed on the return pipe (3) or on the reaction pipe (2);
- two valves (93, 94) placed on either side of said first connection point (91) with one of the valves (94) being placed on the bypass pipe (90); and
- two valves (95, 96) placed on either side of said second connection point (92) with one of the valves (96) being placed on the bypass pipe (90);

and the method comprises a step of manipulating the valves (93, 94, 95, 96) so as to isolate the portion of the reactor (1) located between the first (91) and the second (92) connection points on the reaction pipe (2) side such that the gas/liquid culture medium mixture circulates in the bypass pipe (90) and in the non-isolated portion of the reactor (1) located between the first (91) and the second (92) connection points on the return pipe (3) side.

10. Method according to any one of the preceding claims, in which the method is carried out in a set of photosynthetic reactors (1A; 1B) suitable for culturing photosynthetic microorganisms, comprising at least two reactors (1A; 1B) in accordance with any one of Claims 1 to 12, namely a first reactor (1A) and a second reactor (1B), and comprising at least one linking pipe (71, 72) ensuring a fluidic link between the first reactor (1A) and the second reactor (1B) and at least one valve (77, 78) placed on said linking pipe (71, 72).

11. Method according to Claim 10, in which the set comprises two linking pipes (71, 72) between the two reactors (1A, 1B), each provided with at least one valve (77, 78), including:

- a first linking pipe (71) connecting a point of entry (73) placed on the first reactor (1A) upstream of the circulating means (4) of said first reactor to a point of exit (74) placed on the second reactor (1B) downstream of the circulating means (4) of said second reactor (1B); and
- a second linking pipe (72) connecting a point of entry (75) placed on the second reactor (1B) upstream of the circulating means (4) of said second reactor (1B) to a point of exit (76) placed on the first reactor (1A) downstream of the circulating means (4) of said first reactor (1A).

12. Method according to any one of Claims 1 to 11, in which the controlling step comprises a step of controlling the speed of circulation of the liquid in the reaction pipe (2) between 0.1 and 0.2 m/s in order to establish a two-phase flow regime of the stratified flow type.

13. Method according to any one of Claims 1 to 11, in which the controlling step comprises a step of controlling the speed of circulation of the liquid in the reaction pipe (2) between 0.2 and 1 m/s in order to establish a two-phase flow regime of the pocket or bubble flow type.

14. Method according to any one of the preceding claims, in which the controlling step comprises a step of controlling the speed of circulation of the gas between 0.5 and 0.8 m/s.

15. Method according to any one of the preceding claims, in which the circulating means (4) comprises a propeller (40) driven rotationally by a motor (41) and in which the speed of rotation of the propeller is less than 100 revolutions per minute.

**FIG.1**

**FIG.3**

**FIG.2**

**FIG.4**

EP 2 411 500 B1

FIG.5

EP 2 411 500 B1

**FIG.6a**

**FIG.6b**

**FIG.7a**

**FIG.7b**

FIG.8a

FIG.8b

FIG.8c

FIG.8d

FIG.8e

FIG.8f

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- GB 2118572 A
- ES 2193860 A1
- GB 2331762 A
- ES 2150389 A1
- FR 2685344 A1
- FR 2875511 A3